(19) Europäisches Patentamt
European Patent Office
Office européen des brevets

(11) **EP 4 364 707 A1**

(12) **EUROPEAN PATENT APPLICATION**

(43) Date of publication:
**08.05.2024 Bulletin 2024/19**

(21) Application number: **23206916.1**

(22) Date of filing: **31.10.2023**

(51) International Patent Classification (IPC):
**A61F 13/15** (2006.01)    **A61F 13/49** (2006.01)

(52) Cooperative Patent Classification (CPC):
**A61F 13/15593; A61F 13/15577; A61F 13/15585; A61F 13/15723; A61F 13/15764; A61F 13/49011**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.11.2022 US 202263422467 P**

(71) Applicant: **The Procter & Gamble Company**
**Cincinnati, OH 45202 (US)**

(72) Inventor: **SCHNEIDER, Uwe**
**Cincinnati, 45202 (US)**

(74) Representative: **P&G Patent Germany**
**Procter & Gamble Service GmbH**
**Sulzbacher Straße 40**
**65824 Schwalbach am Taunus (DE)**

(54) **METHOD AND APPARATUS FOR BONDING ELASTIC PARTS UNDER TENSION TO AN ADVANCING CARRIER**

(57)    The present disclosure relates to methods and apparatuses for stretching, transferring, and bonding elastic parts under tension to an advancing carrier substrate during the assembly of absorbent articles. A continuous carrier substrate may be advanced in a machine direction at a first speed, and a discrete elastic part may be cut from a continuous elastic substrate having a direction of stretch in a machine direction. The speed of the discrete elastic part is changed from a second speed to the first speed, and the central region of the discrete elastic part is stretched in the machine direction. The discrete elastic part is bonded with the continuous carrier substrate such that the stretched central region extends in the machine direction between the first and second longitudinal edges of the continuous carrier substrate. The discrete elastic part may also be bonded with the carrier substrate with adhesive and/or mechanical bonds.

Figure 1A

**Description**

CROSS REFERENCE TO RELATED APPLICATION

This application claims the benefit of U.S. Provisional Application No. 63/422,467, filed November 4, 2022, which is incorporated by reference herein in its entirety.

FIELD

The present disclosure relates to methods and apparatuses for manufacturing absorbent articles, and more particularly, to apparatuses and methods for stretching, transferring, and bonding elastic parts to an advancing carrier during the assembly of absorbent articles.

BACKGROUND

Along an assembly line, various types of articles, such as for example, diapers and other absorbent articles, may be assembled by adding components to and/or otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. In some cases, individual components created from an advancing web or webs are combined with other individual components created from other advancing webs. Webs of material and component parts used to manufacture diapers may include: backsheets, topsheets, leg cuffs, waist bands, absorbent core components, front and/or back ears, fastening components, and various types of elastic webs and parts such as leg elastics, barrier leg cuff elastics, stretch side panels, and waist elastics. Once the desired component parts are assembled, the advancing web(s) and component parts are subjected to a final knife cut to separate the web(s) into discrete diapers or other absorbent articles.

Some absorbent articles have components that include elastic parts, such as for example, waistbands. In some configurations, waistbands may be provided as a single layer of elastic material, such as an elastic film. In some configurations, the waistbands may be provided as an elastic laminate that may include elastic material bonded to one or more substrates such as nonwovens, wherein the elastic material may include an elastic film and/or elastic strands. In some assembly operations, the waistbands are joined to an advancing carrier web, such as a continuous topsheet or backsheet web, while the waistbands are in a stretched condition. As such, when the waistbands relax, the carrier web gathers to form corrugations. The resulting laminate is stretchable to the extent that the corrugations allow the waistband to elongate.

When manufacturing absorbent articles, the waistband may be provided as a continuous length of waistband material that may be stretched; cut into discrete waistbands; and bonded with the advancing carrier web while the waistband is in a stretched state. In addition, the carrier web may be advanced in a machine direction and the waistband may be applied to the carrier web such that direction of stretch of the waistband is oriented in a machine direction. For example, some manufacturers may stretch a continuous waistband material in a machine direction and cut the continuous waistband material into stretched discrete waistbands. The stretched discrete waistbands may be turned 90 degrees before placement on and bonding to the advancing carrier web such that direction of stretch is oriented in the cross direction with respect to the carrier web. However, such assembly operations involving the handling and bonding of discrete waistbands in a stretched state can present various challenges.

For example, with reference to a waistband and topsheet bonding operation as an example illustration, adhesive may be applied to either or both the discrete waistband and the topsheet. When applying adhesive to the discrete waistband prior to combining with the topsheet, applied adhesive may migrate from the waistband and contaminate material handling equipment, such as knives, drums, and conveyance devices utilized to place the waistband on the topsheet. Such contaminating adhesive may also migrate to other substrates and components of the assembled article. Instead of applying adhesive to the waistband, adhesive may be applied to the topsheet before combining with the waistband. As such, the adhesive may be applied to the topsheet in discrete patches that are sized to correlate or match with the size of the waistband. Such an operation requires very precise placement of the waistband on the discrete patches of adhesive.

Misplacement of the waistbands on the adhesive may lead to portions of the waistbands being unbonded and may also lead to areas of exposed adhesive. In turn, exposed adhesive that remains tacky can act to unintentionally bond other components together. For example, in subsequent assembly operations, the combined waistband and topsheet may be combined with other advancing substrates and/or components to create discrete absorbent articles that are folded and packaged.

As such, the absorbent article may become bonded to itself in the folded configuration.

In an attempt to avoid the above described negative affects resulting from exposed tacky adhesive in an assembled product, adhesives may be applied in areas that are smaller than the discrete part to be bonded. For example, adhesive may be applied to only central portions of discrete waistband before combining with a topsheet. In another scenario, adhesive may be applied to the topsheet in discrete patches that are relatively smaller than the size of the waistband. In turn, only the central region of a waistband may be bonded with the topsheet. As such, perimeter edges of the waistband may remain unbonded and loose. Such unbonded edges may be aesthetically unpleasing and may lead to undesired tearing and/or separation of the waistband during product use.

In addition, difficulties associated with precisely placing the discrete waistband in a desired location may be exacerbated in assembly processes that require turning the discrete waistbands 90 degrees before combining with an advancing topsheet. Manufacturers may also encounter various difficulties associated with close coupled handovers of stretched waistbands between conveyance devices while maintaining the stretched condition of the waistbands. Further, components of the apparatuses associated with waistband application processes may be relatively inflexible with respect to making absorbent articles of different sizes. For example, existing assembly operations may be configured to place waistbands at fixed pitch distances that cannot be altered without changing several apparatus components when needed to make articles of different sizes that require changes in pitch distances.

Consequently, it would be beneficial to provide methods and apparatuses for bonding waistbands to carrier webs while helping to increase the size of bonded areas and reducing the chances of having exposed adhesives during subsequent assembly operations; providing flexibility to accommodate desired speed and/or pitch changes; and/or improving abilities to transfer stretched waistbands between close coupled conveyance devices while helping to maintain the stretched condition of the waistbands.

SUMMARY

In one form, a method of assembling absorbent articles comprises steps of: advancing a carrier substrate at a first speed in a machine direction, the carrier substrate comprising a first longitudinal edge and a second longitudinal edge separated from the first longitudinal edge in a cross direction; advancing a continuous elastic substrate at a second speed in the machine direction, the continuous elastic substrate comprising a first longitudinal edge and a second longitudinal edge separated from the first longitudinal edge in the cross direction, wherein the continuous elastic substrate is stretchable in the machine direction; cutting an elastic part from the continuous elastic substrate, the elastic part comprising a first end region and a second end region separated from the first end region in the machine direction by a central region; changing a speed of the elastic part from the second speed to the first speed; stretching the central region of the discrete elastic part in the machine direction; rotating the discrete elastic part such that the second end region is separated from the first end region in the cross direction by the central region; positioning the elastic part on the carrier substrate such that the stretched central region extends in the cross direction between the first and second longitudinal edges of the carrier substrate; adhesively bonding the stretched central region of the elastic part with the carrier substrate; and mechanically bonding the first end region and the second end region of the elastic part with the carrier substrate.

In another form, a method of assembling absorbent articles comprises steps of: providing an elastic part comprising a first surface and an opposing second surface, the elastic part further comprising a first end region and a second end region separated from the first end region in a machine direction by a central region; providing a zone of adhesive positioned on the second surface of the elastic part; advancing the elastic part in a machine direction on a first roll, where the second surface is facing radially outward; stretching the central region of the elastic part in the machine direction; transferring the first end region and the second end region of the elastic part from the first roll to a rotatable transfer device, where the second surface of the elastic part is facing radially inward; rotating the discrete elastic part such that the second end region is separated from the first end region in the cross direction by the central region; transferring the stretched elastic part to a second roll, where the second surface of the elastic part is facing radially outward; advancing a carrier substrate adjacent the second roll, the carrier substrate comprising a first longitudinal edge and a second longitudinal edge separated from the first longitudinal edge in a cross direction; advancing the elastic part from the second roll to the carrier substrate such that the stretched central region extends in the cross direction between the first and second longitudinal edges of the carrier substrate; adhesively bonding the stretched central region of the elastic part with the carrier substrate; and mechanically bonding the first end region and the second end region of the elastic part with the carrier substrate.

In yet another form, a method of assembling absorbent articles comprises steps of: advancing a carrier substrate at a first speed in a machine direction, the carrier substrate comprising a first longitudinal edge and a second longitudinal edge separated from the first longitudinal edge in a cross direction; advancing a continuous elastic substrate at a second speed in the machine direction, the continuous elastic substrate comprising a first longitudinal edge and a second longitudinal edge separated from the first longitudinal edge in the cross direction, wherein the continuous elastic substrate is stretchable in the machine direction; cutting an elastic part from the continuous elastic substrate, the elastic part comprising a first end region and a second end region separated from the first end region in the machine direction by a central region; changing a speed of the elastic part from the second speed to the first speed; stretching the central region of the discrete elastic part in the machine direction; rotating the stretched central region such that the stretched central region is oriented in the cross direction; positioning the elastic part on the carrier substrate such that the stretched central region extends in the cross direction between the first and second longitudinal edges of the carrier substrate; and dividing the elastic part into a first waistband and a second waistband by cutting the carrier substrate along the cross direction through the elastic part.

Various embodiments solve the above-mentioned problems and provide methods and devices useful for

These and other features, aspects, and advantages of various embodiments will become better understood with reference to the following description, figures, and claims.

BRIEF DESCRIPTION OF THE DRAWINGS

Many aspects of this disclosure can be better understood with reference to the following figures.

FIG. 1A is a partially cut away plan view of an absorbent article in the form of a taped diaper that may include one or more substrates bonded in accordance with the present disclosure with the portion of the diaper that faces away from a wearer oriented towards the viewer.

FIG. 1B is a plan view of the absorbent article of FIG. 1A that may include one or more substrates bonded in accordance with the present disclosure with the portion of the diaper that faces toward a wearer oriented towards the viewer.

FIG. 2 is a schematic side view of an apparatus for bonding elastic parts to an advancing carrier web.

FIG. 2A is a detailed schematic view of a bonding apparatus with a pressing surface comprising an ultrasonic bonding device.

FIG. 2B is a schematic side view of an apparatus for bonding elastic parts to an advancing carrier web.

FIG. 2C is a schematic side view of an apparatus for bonding elastic parts to an advancing carrier web.

FIG. 3 is a view of a carrier substrate taken along section 3-3 in FIG. 2.

FIG. 3A is a view of a carrier substrate with leg cuffs taken along section 3-3 in FIG. 2.

FIG. 4 is a view of a continuous elastic substrate taken along section 4-4 in FIG. 2.

FIG. 4A is a view of a continuous elastic substrate with corrugations and corrugation lines taken along section 4-4 in FIG. 2.

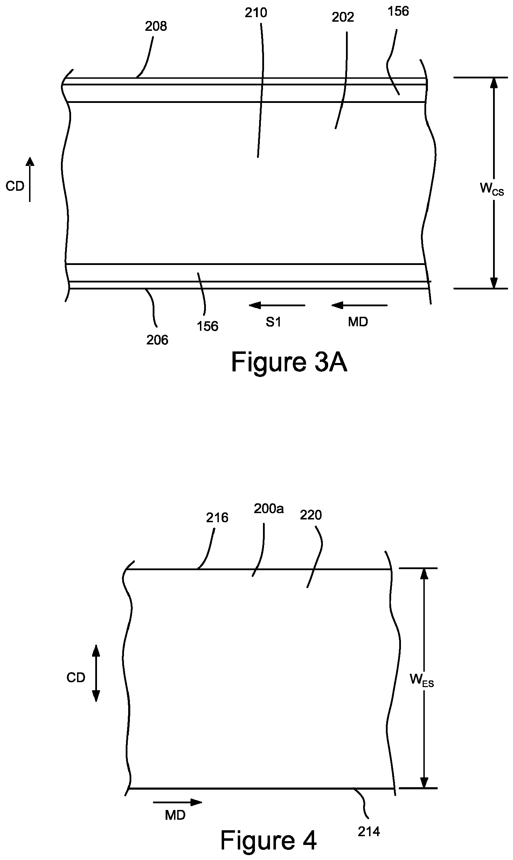

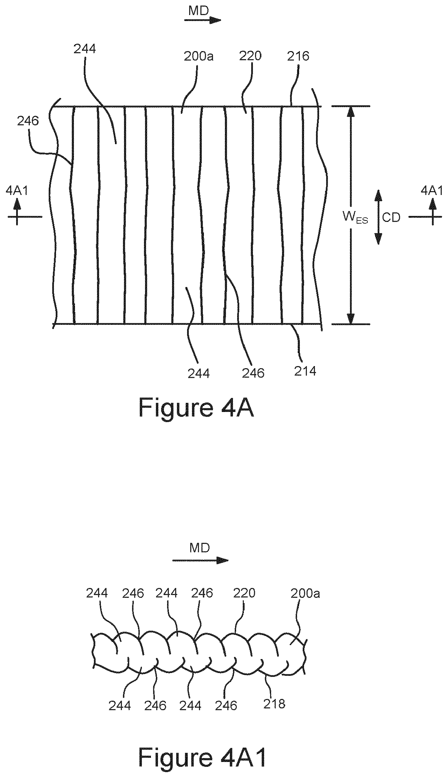

FIG. 4A1 is a view of the continuous elastic substrate taken along section 4A1-4A1 in FIG. 4A.

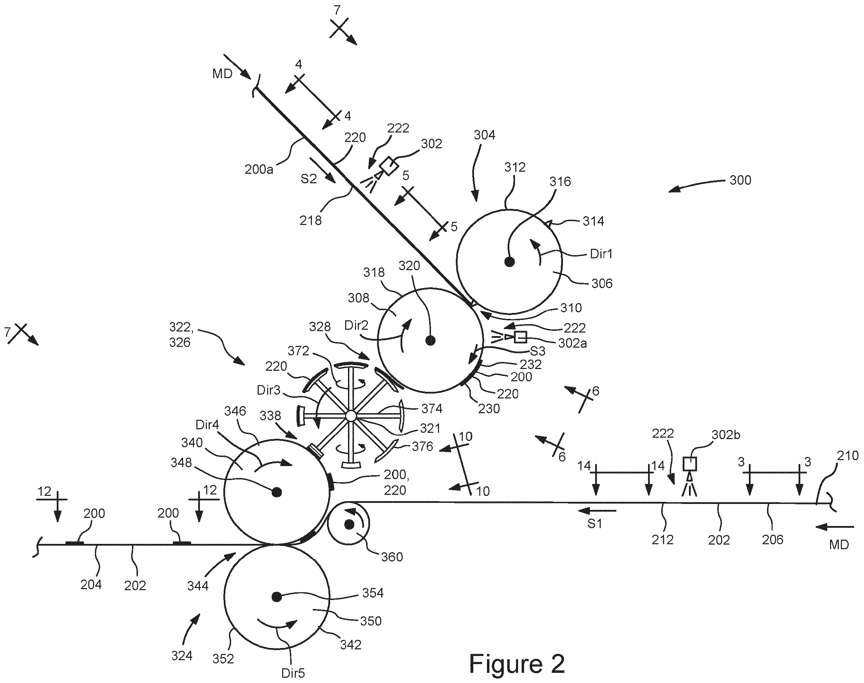

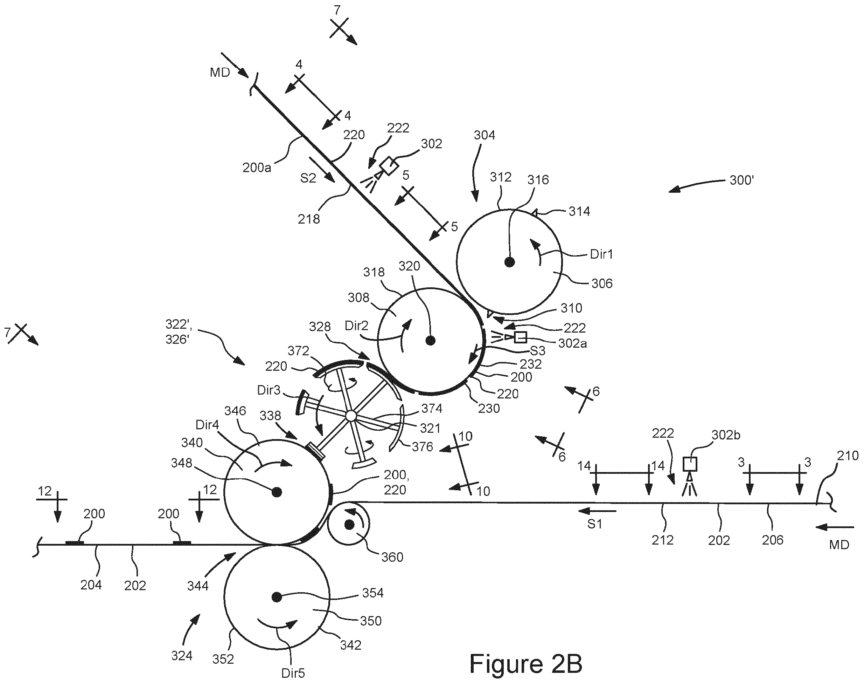

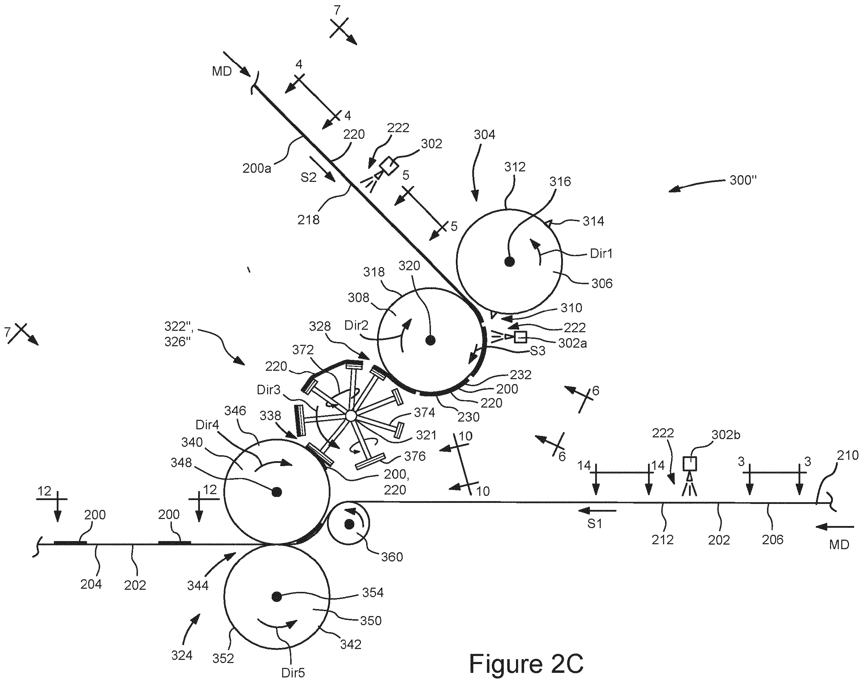

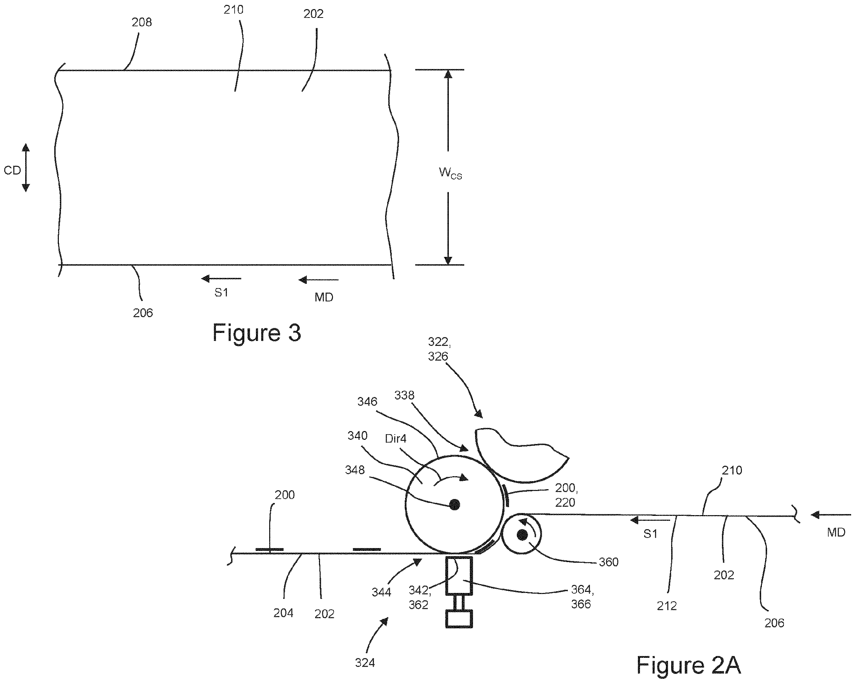

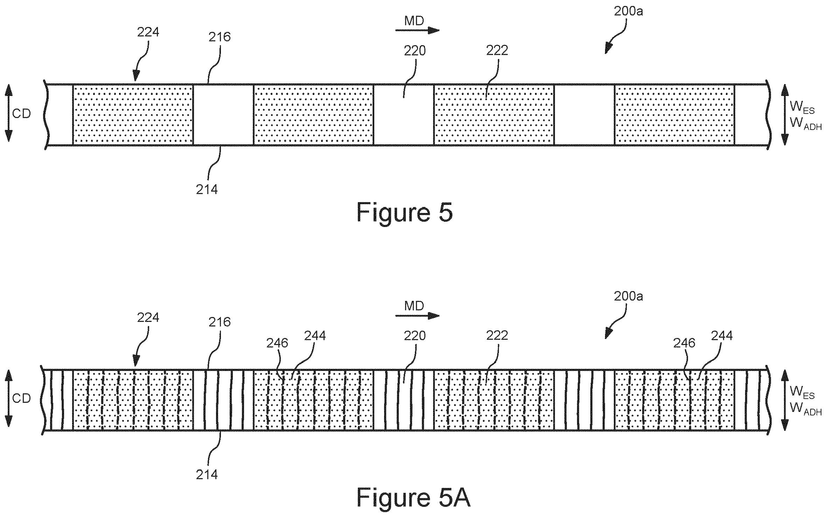

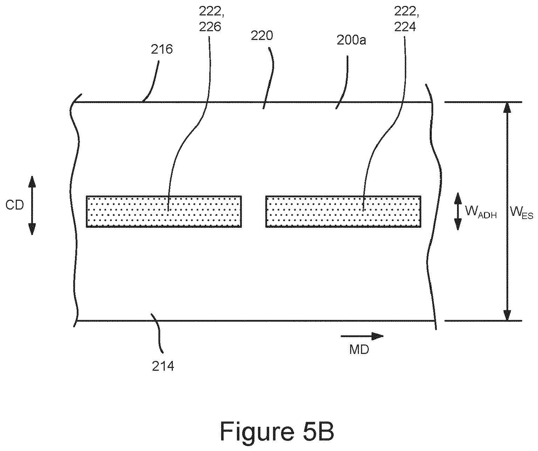

FIG. 5 is a view of a continuous elastic substrate with a continuous region of adhesive taken along section 5-5 in FIG. 2.

FIG. 5A is a view of a continuous elastic substrate with a continuous region of adhesive covering corrugations taken along section 5-5 in FIG. 2.

FIG. 5B is a view of a continuous elastic substrate with discrete patches of adhesive taken along section 5-5 in FIG. 2.

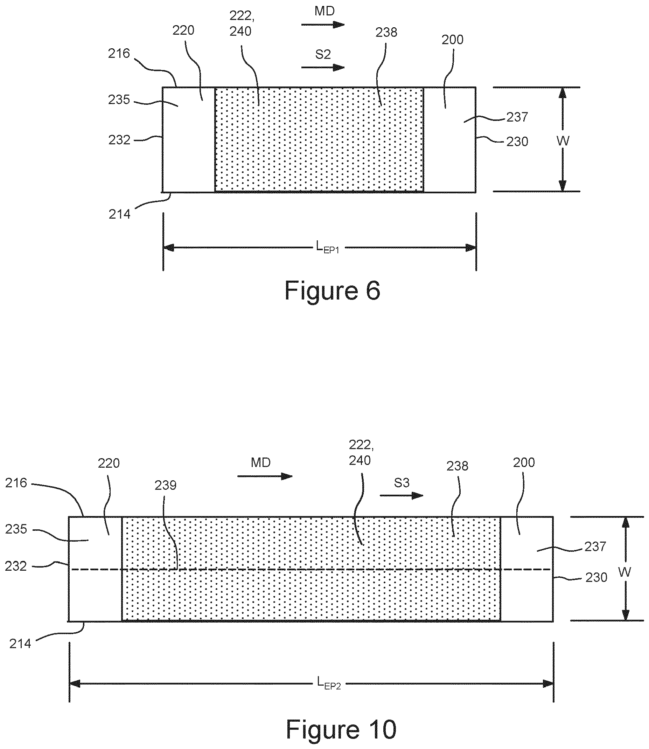

FIG. 6 is a view of a discrete elastic part laid out flat with a zone of adhesive thereon taken along section 6-6- in FIG. 2.

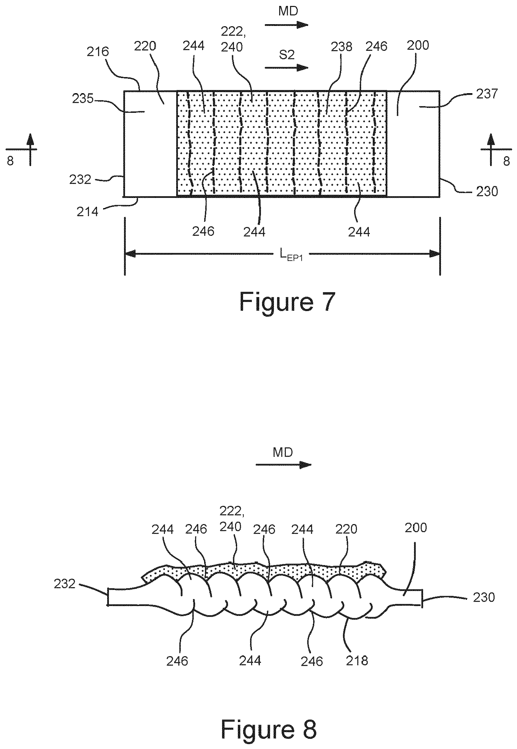

FIG. 7 is a view of a discrete elastic part laid out flat with corrugations, corrugation lines, and a zone of adhesive taken along section 6-6 in FIG. 2.

FIG. 8 is a view of the discrete elastic part taken along section 8-8 in FIG. 7.

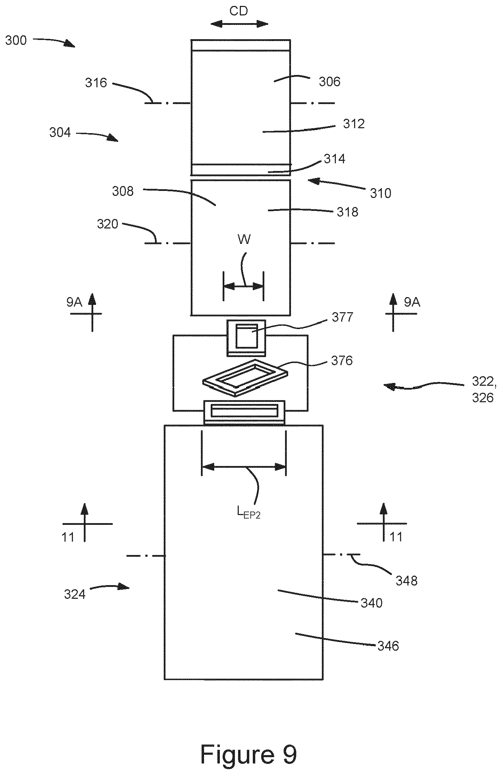

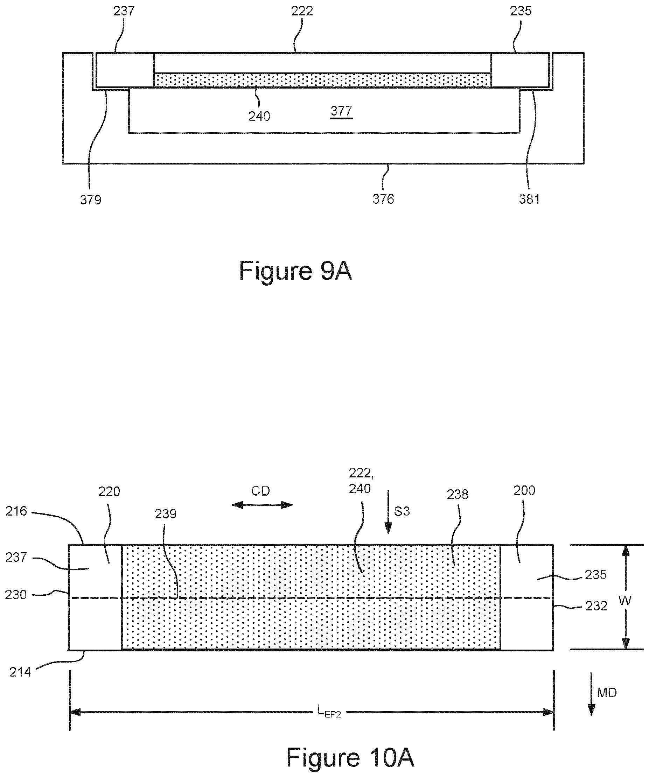

FIG. 9 is a view of a cutting device, transfer device, and bonding device taken along section 7-7 in FIG. 2.

FIG. 9A is a cross-sectional side view of the recess defined by the plate in FIG. 9 taken along section 9A-9A in FIG. 9.

FIG. 10 is a view of a stretched discrete elastic part laid out flat with a zone of adhesive thereon taken along section 6-6 in FIG. 2.

FIG. 10A is a view of a stretched discrete elastic part laid out flat with the zone of adhesive taken along section 10-10 in FIG. 2.

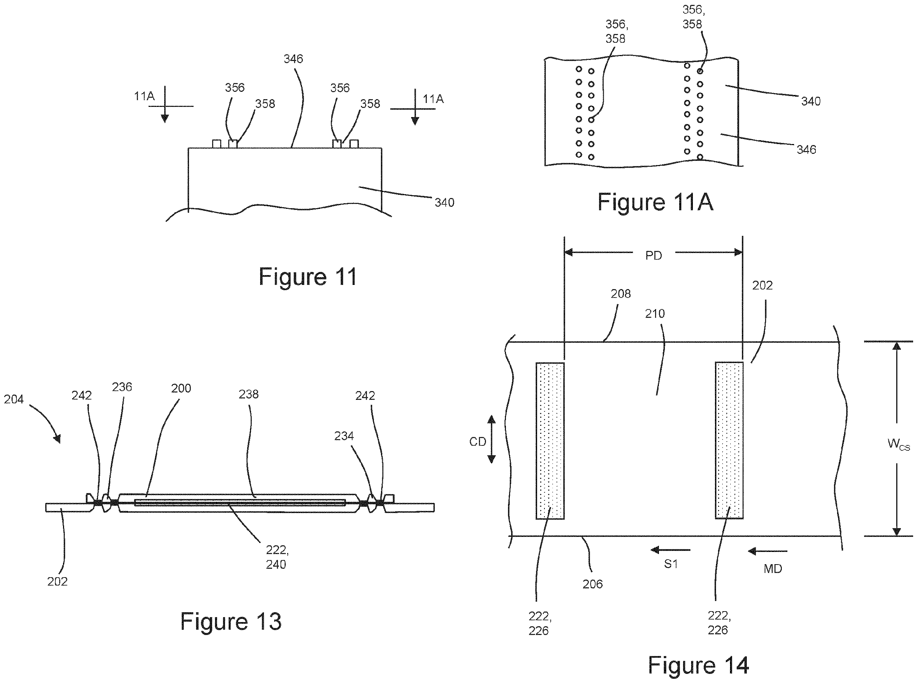

FIG. 11 is a detailed cross sectional view of a pattern roll from FIG. 9 showing bonding elements extending radially outward from an outer circumferential surface taken along line 11-11.

FIG. 11A is a detailed view of a portion of the outer circumferential surface of the pattern roll showing bonding elements from FIG. 11 taken along line 11A-11A.

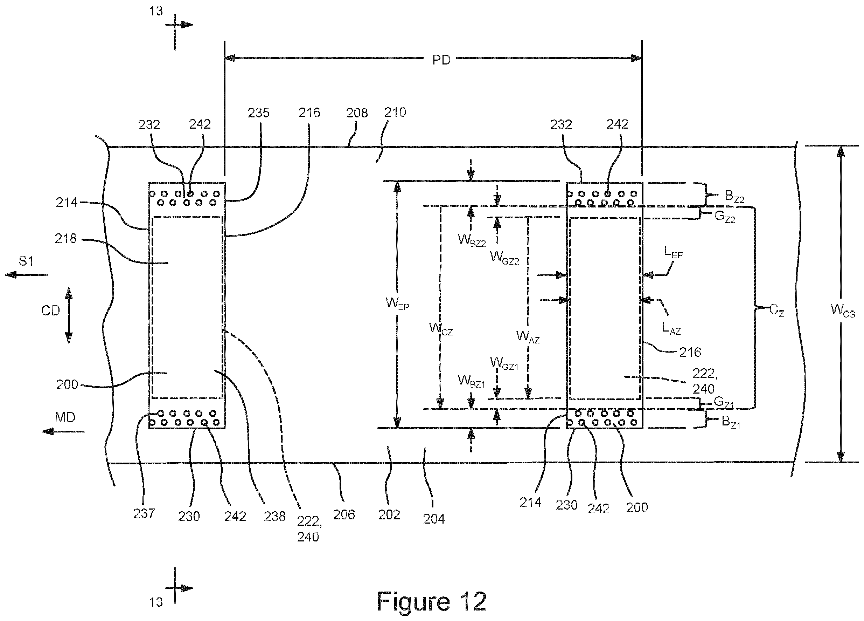

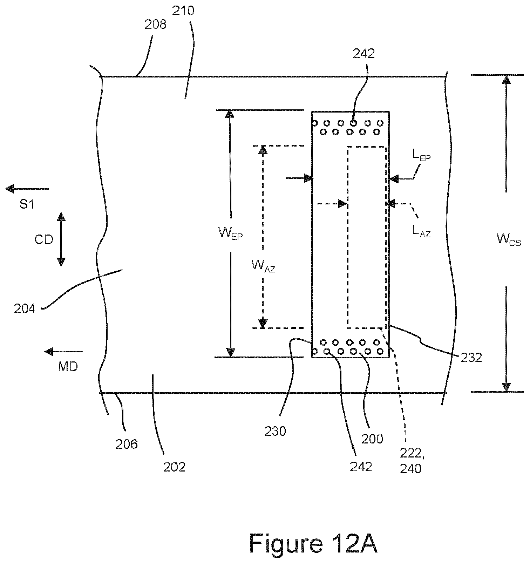

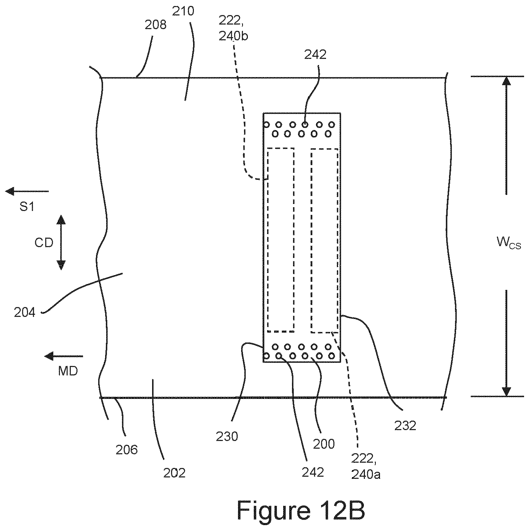

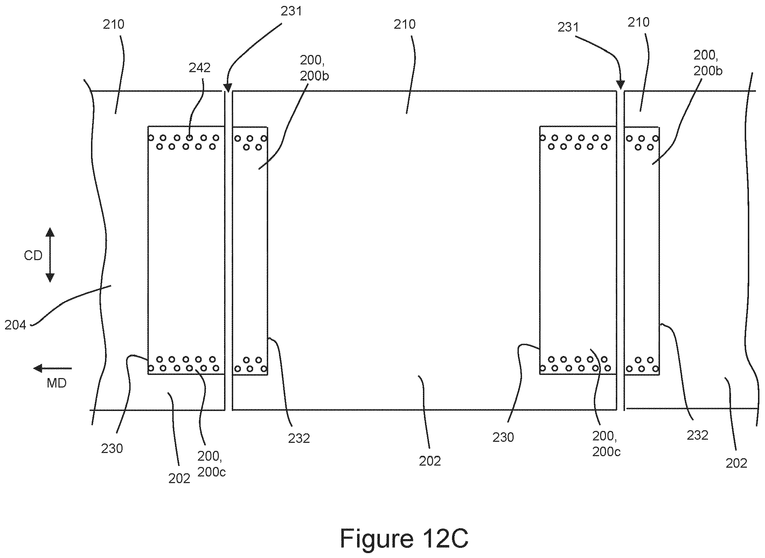

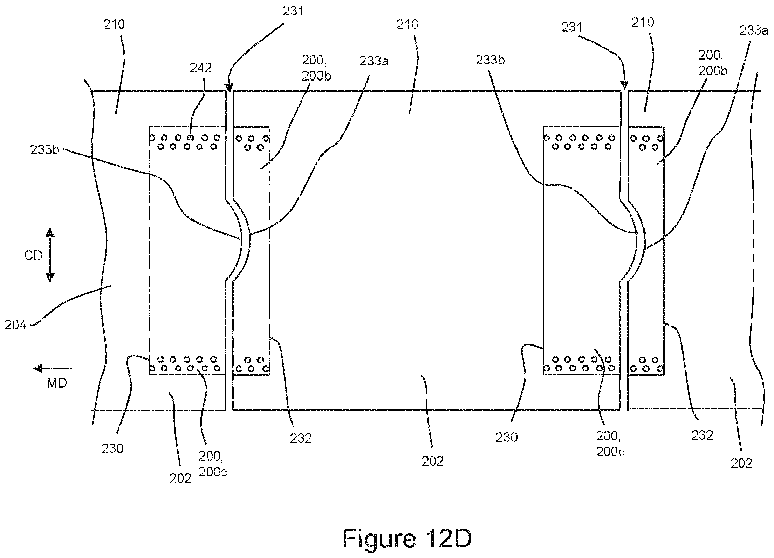

FIG. 12 is a view of a laminate including the elastic part and the carrier substrate taken along section 12-12 in FIG. 2.

FIG. 12A is a view of the laminate including the elastic part and the carrier substrate with a zone of adhesive that extends for a length less than a length of the elastic part.

FIG. 12B is a view of the laminate including the elastic part and the carrier substrate with a plurality of zones of adhesive.

FIG. 12C is a view of the laminate of FIG. 12 including the elastic part and the carrier substrate after being subjected to a final knife cut operation that applies cut lines through the carrier substrate and discrete elastic parts.

FIG. 12D is a view of the laminate of FIG. 12 including the elastic part and the carrier substrate after being subjected to a final knife cut operation that applies curved cut lines through the carrier substrate and discrete elastic parts.

FIG. 13 is a view of the laminate including the elastic part and the carrier substrate taken along section 13-13 in FIG. 12.
FIG. 14 is a view of the carrier substrate and adhesive taken along section 14-14 in FIG. 2.

It should be understood that the various embodiments are not limited to the examples illustrated in the figures.

DETAILED DESCRIPTION

The following term explanations may be useful in understanding the present disclosure: "Absorbent article" is used herein to refer to consumer products whose primary function is to absorb and retain soils and wastes. Absorbent articles can comprise sanitary napkins, tampons, panty liners, interlabial devices, wound dressings, wipes, disposable diapers including taped diapers and diaper pants, inserts for diapers with a reusable outer cover, adult incontinent diapers, adult incontinent pads, and adult incontinent pants. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

The term "feminine hygiene articles" refers to disposable absorbent articles used by women for catamenial protection. Such feminine hygiene articles may include sanitary napkins, tampons, interlabial products, incontinence devices, and pantiliners. Non-limiting examples of panty liners and sanitary napkins include those disclosed in U.S. Pat. Nos. 4,324,246; 4,463,045; 4,342,314; 4,556,146; 4,589,876; 4,687,478; 4,950,264; 5,009,653; 5,267,992; and 6,004,893, which are all incorporated by reference herein.

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.

"Consolidation," "consolidating," and "consolidated" refers to a material undergoing a reduction in elongation from a first stretched length to a second stretched length that is less than the first stretched length and greater than zero.

"Relaxed state" defines a length of material when not stretched by an applied force.

In the context of the present description, an elongation of 0% refers to a material in relaxed state having a relaxed length of L, and elongation of 150% represents $2.5\times$ the relaxed length, L, of the material. For example, an elastic film having a relaxed length of 100 millimeters would have a length of 250 millimeters at 150% elongation. And an elastic film having a relaxed length of 100 millimeters would have a length of 180 millimeters at 80% elongation.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

The term "substrate" is used herein to describe a material which is primarily two-dimensional (i.e., in an XY plane) and whose thickness (in a Z direction) is relatively small (i.e., 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. In some configurations, a nonwoven may comprise a polyolefin based nonwoven, including but not limited to nonwovens having polypropylene fibers and/or polyethylene fibers and/or bicomponent fibers comprising a polyolefin. Nonlimiting examples of suitable fibers include spunbond, spunlaid, meltblown, spunmelt, solvent-spun, electrospun, carded, film fibrillated, melt-film fibrillated, air-laid, dry-laid, wet-laid staple fibers, and other nonwoven web materials formed in part or in whole of polymer fibers as known in the art, and workable combinations thereof. Nonwovens do not have a woven or knitted filament pattern. It is to be appreciated that nonwovens having various basis weights can be used in accordance with the methods herein. For example, some nonwovens may have a basis weight of at least about 8 gsm, 12 gsm, 16 gsm, 20 gsm, 25 gsm, 30 gsm, 40 gsm, or 65 gsm. Some nonwovens may have basis weight of about 8 gsm to about 65 gsm, specifically reciting all 1 gsm increments within the above-recited ranges and all ranges formed therein or thereby.

It is to be appreciated that films having various basis weights can be used in accordance with the methods herein. For example, some films may have a basis weight of at least about 8 gsm, 12 gsm, 16 gsm, 20 gsm, 25 gsm, 30 gsm, 40 gsm, or 60 gsm. Some films may have basis weight of about 5 gsm to about 150 gsm, specifically reciting all 1 gsm increments within the above-recited ranges and all ranges formed therein or thereby.

It is to be appreciated that elastic films discussed herein may comprise various materials and/or components. Some elastomeric compositions may comprise thermoplastic elastomers selected from the group consisting of Styrenic block copolymers, poly-esters, polyurethanes, polyether amides, and combinations thereof. Suitable styrenic block copolymers may be diblock, triblock, tetrablock, or other multi-block copolymers having at least one styrenic block. Exemplary styrenic block copolymers include styrene-butadiene-styrene, styrene-isoprene-styrene, styrene-ethylene/butylenes-styrene, styrene-ethylene/propylene-styrene, and the like. Commercially available styrenic block copolymers include KRATON (styrenic block copolymer; available from the Kraton Chemical Company, Houston, Tex.), SEPTON (styrenic block copolymer; available from Kuraray America, Inc., New York, N.Y.), VECTOR (styrenic block copolymer; available from TSRC Dexco Chemical Company, Houston, Tex.) can be used. Additional commercially available elastomers include ESTANE (polyurethane; available from Lubrizol, Inc, Ohio), PEBAX (polyether block amide; available from Arkema Chemicals, Philadelphia, Pa.), and HYTREL (polyester; available from DuPont, Wilmington, Del.).

Semi-crystalline, or metallocene polyolefins may be used in disposable absorbent products. The polyolefin elastomer materials herein may include, but are not limited to, any polymers or copolymers of polyolefins such as polyethylene and polypropylene. Examples of elastomeric polypropylenes include an elastic random poly(propylene/olefin) copolymer, an isotactic polypropylene containing stereo-irregularity, an isotactic/atactic polypropylene block copolymer, an isotactic polypropylene/random poly(propylene/olefin) copolymer block copolymer, a stereoblock elastomeric polypropylene, a syndiotactic polypropylene block poly(ethylene-co-propylene) block syndiotactic polypropylene triblock copolymer, an isotactic polypropylene block regioirregular polypropylene block isotactic polypropylene triblock copolymer, a polyethylene random (ethylene/olefin) copolymer block copolymer, a reactor blend polypropylene, a very low density polypropylene (or, equivalently, ultra low density polypropylene), a metallocene polypropylene, and blends or combinations thereof. Some homopolyolefins and random copolymers, as well as blends of such random copolymers, known by tradenames Vistamaxx™ available from ExxonMobil and VERSIFY™ from Dow, tend to show elastic performance. In some embodiments, two or more elastomers may be blended to achieve the desired elastic performance. For example, Styrenic block copolymer can be blended with polyolefin based elastomers, or polypropylene based elastomer can be blended with other polyolefin based elastomers.

In some configurations, elastic films discussed herein may comprise a fluorophore that excites to higher energy by absorbing photons (light) at relatively lower wavelengths, and then emits photons (light) at relatively higher wavelengths. An optical brightener is an example of fluorophore material, and an optical brightener may be configured to emit light in the blue color wavelength region. In some configurations fluorophores or optical brighteners can be added to the elastic film or polymeric material in the range of 5 ppm to almost 2%. Depending on the material and need, the amount of additive can vary. Examples of optical brightener that can be used in polymeric materials are Benetex OB series available from Mayzo, Ga., USA. By changing the fluorophore material, the wavelength of absorption and emission can be changed. Depending on the emission wavelength (at maximum intensity), a vision system may be configured to detect such emissions. For example, a vision system designed to detect the higher wavelength blue light can be used to identify a component containing an optical brightener. In turn, such vision systems may be adapted to identify material containing fluorophore (optical brightener) from the other materials without it. As such, the vision systems may be used for detection, rejection, and/or accuracy of material placement during assembly processes. Depending on the emission wavelength (color), different camera light filters can be used to detect materials.

Components of the disposable absorbent articles (i.e., diaper, disposable pant, adult incontinence article, sanitary napkin, pantiliner, etc.) described in this specification can at least partially be comprised of bio-sourced content as described in US 2007/0219521 A1 Hird et al published on Sep. 20, 2007, US 2011/0139658 A1 Hird et al published on Jun. 16, 2011, US 2011/0139657 A1 Hird et al published on Jun. 16, 2011, US 2011/0152812 A1 Hird et al published on Jun. 23, 2011, US 2011/0139662 A1 Hird et al published on Jun. 16, 2011, and US 2011/0139659 A1 Hird et al published on Jun. 16, 2011. These components include, but are not limited to, topsheet nonwovens, backsheet films, backsheet nonwovens, side panel nonwovens, barrier leg cuff nonwovens, super absorbent, nonwoven acquisition layers, core wrap nonwovens, adhesives, fastener hooks, and fastener landing zone nonwovens and film bases. In at least one embodiment, a disposable absorbent article component comprises a bio-based content value from about 10% to about 100% using ASTM D6866-10, method B, in another embodiment, from about 25% to about 75%, and in yet another embodiment, from about 50% to about 60% using ASTM D6866-10, method B. In order to apply the methodology of ASTM D6866-10 to determine the bio-based content of any disposable absorbent article component, a representative sample of the disposable absorbent article component must be obtained for testing. In at least one embodiment, the disposable absorbent article component can be ground into particulates less than about 20 mesh using known grinding methods (e.g., Wiley® mill), and a representative sample of suitable mass taken from the randomly mixed particles.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

Aspects of the present disclosure relate to methods and apparatuses for bonding substrates used in absorbent articles, and in particular, methods and apparatuses for stretching, transferring, and bonding elastic parts under tension to an advancing carrier substrate during the assembly of absorbent articles. With regard to the assembly processes described herein, a continuous carrier substrate may be advanced in a machine direction at a first speed, the carrier substrate comprising a first longitudinal edge and a second longitudinal edge separated from the first longitudinal side in a cross direction. A continuous elastic substrate may also be advanced in the machine direction at a second speed with at least one direction of stretch extending in the machine direction, wherein the second speed is slower than the first speed. A discrete elastic part is separated from the continuous elastic substrate, wherein the discrete elastic part comprises a first end region and a second end region separated from the first end region in the machine direction by a central region. The speed of the discrete elastic part is changed from the second speed to the first speed, and the central region of the discrete elastic part is stretched in the machine direction. The discrete elastic part is rotated so that the stretched central region is aligned in the cross direction. The discrete elastic part is then bonded with the continuous carrier substrate such that the stretched central region extends in the cross direction between the first and second longitudinal edges of the continuous carrier substrate.

As discussed in more detail below, the discrete elastic parts are cut from a continuous elastic substrate having a direction of stretch in the machine direction, after which the discrete elastic part undergoes a 90 degree turn operation before bonding with the carrier substrate. The methods and apparatuses herein also provide the ability to bond the discrete elastic part with the carrier substrate with adhesive and/or mechanical bonds. In some configurations, adhesive may be applied so as to help maximize bonded areas between the elastic part and the carrier substrate while mechanical bonds may also be used to help reduce perimeter edges of the elastic part that may otherwise remain unbonded and loose. Consecutively arranged conveying components of the apparatus may also be configured with intermeshing nubs and bonding elements and/or contoured outer rims that help improve abilities to transfer of discrete elastic parts while maintaining the elastic parts in a stretched condition. In addition, conveying components may also be configured with variable rotational velocities that help provide operational flexibility by reducing the necessity to change components to accommodate desired changes in speeds and/or pitching in manufacturing operations.

It is to be appreciated that the systems and methods disclosed herein are applicable to work with various types of converting processes and/or machines, such as for example, absorbent article manufacturing and assembly processes. The methods and apparatuses are discussed below in the context of manufacturing diapers that may be configured as taped diapers or pant diapers.

The term "taped diaper" (also referred to as "open diaper") refers to disposable absorbent articles having an initial front waist region and an initial back waist region that are not fastened, pre-fastened, or connected to each other as packaged, prior to being applied to the wearer. A taped diaper may be folded about the lateral centerline with the interior of one waist region in surface to surface contact with the interior of the opposing waist region without fastening or joining the waist regions together. Example taped diapers are disclosed in various suitable configurations U.S. Pat. Nos. 5,167,897, 5,360,420, 5,599,335, 5,643,588, 5,674,216, 5,702,551, 5,968,025, 6,107,537, 6,118,041, 6,153,209, 6,410,129, 6,426,444, 6,586,652, 6,627,787, 6,617,016, 6,825,393, and 6,861,571; and U.S. Patent Publication Nos. 2013/0072887 A1; 2013/0211356 A1; and 2013/0306226 A1, which are all incorporated by reference herein.

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed or pre-fastened by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed). Example diaper pants in various configurations are disclosed in U.S. Pat. Nos. 4,940,464; 5,092,861; 5,246,433; 5,569,234; 5,897,545; 5,957,908; 6,120,487; 6,120,489; 7,569,039 and U.S. Patent Publication Nos. 2003/0233082 A1; 2005/0107764 A1, 2012/0061016 A1, 2012/0061015 A1; 2013/0255861 A1; 2013/0255862 A1; 2013/0255863 A1; 2013/0255864 A1; and 2013/0255865 A1, all of which are incorporated by reference herein.

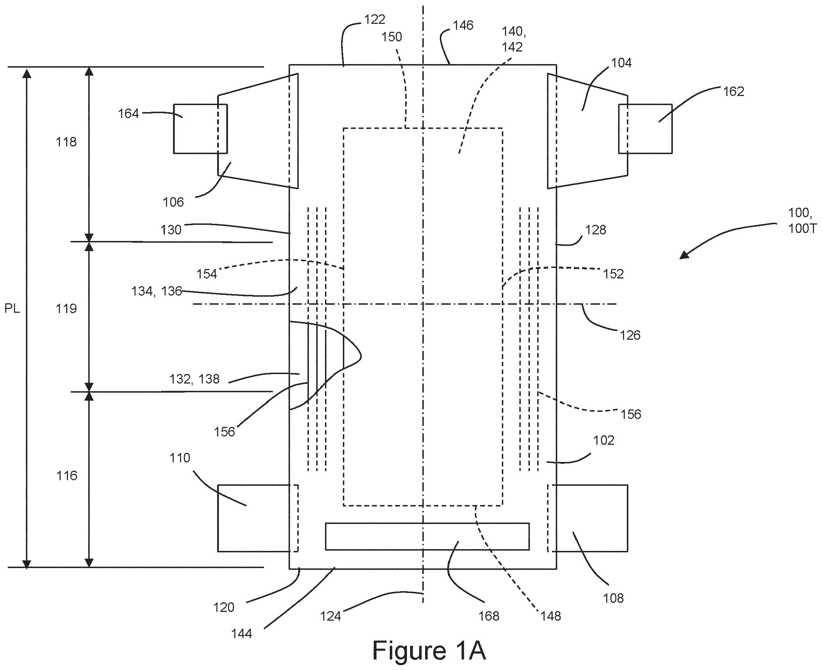

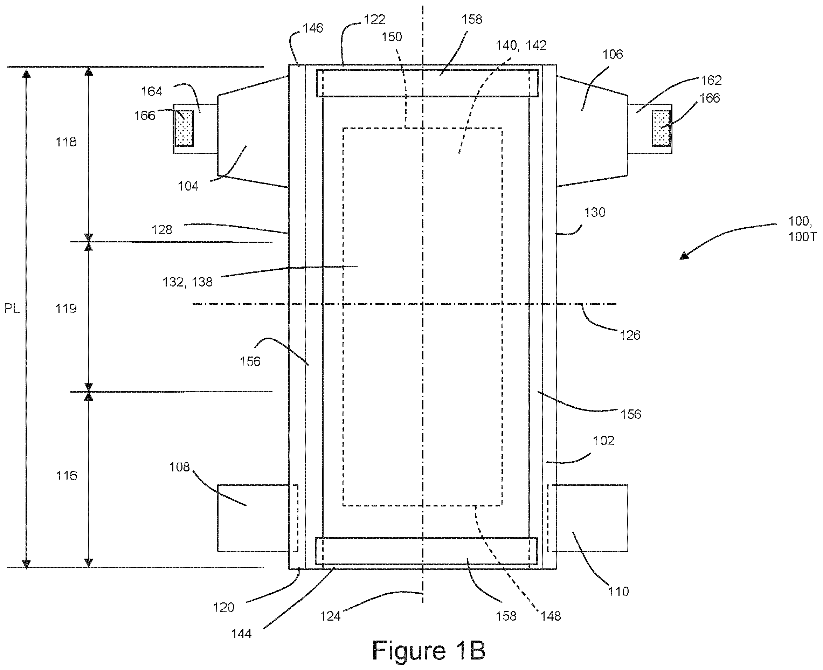

For the purposes of a specific illustration, FIGS. 1A and 1B show an example of an absorbent article 100 that may be assembled in accordance with the methods and apparatuses disclosed herein. In particular, FIG. 1A shows one example of a plan view of an absorbent article 100 configured as a taped diaper 100T, with the portion of the diaper that faces away from a wearer oriented towards the viewer. And FIG. 1B shows a plan view of the diaper 100 with the portion of the diaper that faces toward a wearer oriented towards the viewer. The taped diaper 100T shown in FIGS. 1A and 1B includes an absorbent chassis 102, first and second rear side panels 104 and 106; and first and second front side panels 108 and 110.

As shown in FIGS. 1A and 1B, the diaper 100 and the chassis 102 each include a first waist region 116, a second waist region 118, and a crotch region 119 disposed intermediate the first and second waist regions. The first waist region 116 may be configured as a front waist region, and the second waist region 118 may be configured as a back waist region. In some embodiments, the length of each of the front waist region, back waist region, and crotch region may be ⅓ of the length of the absorbent article 100. The absorbent article may also include a laterally extending front waist edge 120 in the front waist region 116 and a longitudinally opposing and laterally extending back waist edge 122 in the back waist region 118. To provide a frame of reference for the present discussion, the diaper 100T in FIGS. 1A and 1B is shown with a longitudinal axis 124 and a lateral axis 126. The longitudinal axis 124 may extend through a midpoint of the front waist edge 120 and through a midpoint of the back waist edge 122. And the lateral axis 126 may extend through a midpoint of a first longitudinal or right side edge 128 and through a midpoint of a second longitudinal or left side edge 130.

As shown in FIGS. 1A and 1B, the diaper 100 includes an inner, wearer facing surface 132, and an outer, garment facing surface 134. As such, it is also to be appreciated that the various components of the diaper described below may each include inner, wearer facing surfaces 132, and an outer, garment facing surfaces 134. The chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the diaper 100 may also include other features, such as leg elastics and/or leg cuffs, an elastic waist region, and/or flaps, e.g., side panels and/or ears, to enhance the fits around the legs and waist of the wearer, to enhance the fit around the legs of the wearer.

As shown in FIGS. 1A and 1B, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in FIG. 1A, the laterally extending end edges 144 and 146 may form a portion of the laterally extending front waist edge 120 in the front waist region 116 and a portion of the longitudinally opposing and laterally extending back waist edge 122 in the back waist region 118. The distance between the first lateral end edge 144 and the second lateral end edge 146 may define a pitch length, PL, of the chassis 102. When the diaper 100 is worn on the lower torso of a wearer, the front waist edge 120 and the back waist edge 122 may encircle a portion of the waist of the wearer. At the same time, the side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

It is to also be appreciated that a portion or the whole of the diaper 100 may also be made laterally extensible. The additional extensibility may help allow the diaper 100 to conform to the body of a wearer during movement by the wearer. The additional extensibility may also help, for example, the user of the diaper 100, including a chassis 102 having a particular size before extension, to extend the front waist region 116, the back waist region 118, or both waist regions of the diaper 100 and/or chassis 102 to provide additional body coverage for wearers of differing size, i.e., to tailor the diaper to an individual wearer. Such extension of the waist region or regions may give the absorbent article a generally hourglass shape, so long as the crotch region is extended to a relatively lesser degree than the waist region or regions, and may impart a tailored appearance to the article when it is worn.

As previously mentioned, the diaper 100 may include a backsheet 136. The backsheet 136 may also define the outer surface 134 of the chassis 102. The backsheet 136 may be impervious to fluids (e.g., menses, urine, and/or runny feces) and may be manufactured in part from a thin plastic film, although other flexible liquid impervious materials may also be used. The backsheet 136 may prevent the exudates absorbed and contained in the absorbent core from wetting articles which contact the diaper 100, such as bedsheets, pajamas and undergarments. The backsheet 136 may also comprise a woven or nonwoven material, polymeric films such as thermoplastic films of polyethylene or polypropylene, and/or a multi-layer or composite materials comprising a film and a nonwoven material (e.g., having an inner film layer and an outer nonwoven layer). The backsheet may also comprise an elastomeric film. An example backsheet 136 may be a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet 136 may also be embossed and/or matte-finished to provide a more clothlike appearance. Further, the backsheet 136 may permit vapors to escape from the absorbent core (i.e., the backsheet is breathable) while still preventing exudates from passing through the backsheet 136. The size of the backsheet 136 may be dictated by the size of the absorbent core 142 and/or particular configuration or size of the diaper 100.

Also described above, the diaper 100 may include a topsheet 138. The topsheet 138 may also define all or part of the inner surface 132 of the chassis 102. The topsheet 138 may be compliant, soft feeling, and non-irritating to the wearer's skin. It may be elastically stretchable in one or two directions. Further, the topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art.

Topsheets 138 may be selected from high loft nonwoven topsheets, apertured film topsheets and apertured nonwoven topsheets. Apertured film topsheets may be pervious to bodily exudates, yet substantially non-absorbent, and have a reduced tendency to allow fluids to pass back through and rewet the wearer's skin. Exemplary apertured films may include those described in U.S. Pat. Nos. 5,628,097; 5,916,661; 6,545,197; and 6,107,539, which are all incorporated by reference herein.

As mentioned above, the diaper 100 may also include an absorbent assembly 140 that is joined to the chassis 102. As shown in FIGS. 1A and 1B, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Pat. Nos. 4,610,678; 4,673,402; 4,888,231; and 4,834,735, which are all incorporated by reference herein.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprise primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Pat. Nos. 5,599,335; 5,562,646; 5,669,894; and 6,790,798 as well as U.S. Patent Publication Nos. 2004/0158212 A1 and 2004/0097895 A1.

As previously mentioned, the diaper 100 may also include elasticized leg cuffs 156 and an elasticized waistband 158. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Pat. Nos. 3,860,003; 4,909,803; 4,695,278; 4,795,454; 4,704,115; and U.S. Patent Publication No. 2009/0312730 A1, which are all incorporated by reference herein.

As shown in FIG. 1B, the chassis 102 may include longitudinally extending and laterally opposing leg cuffs 156 that are disposed on the interior surface 132 of the chassis 102 that faces inwardly toward the wearer and contacts the wearer. Each leg cuff may have a proximal edge. The leg cuffs may also overlap the absorbent assembly 140, wherein the proximal edges extend laterally inward of the respective side edges of the absorbent assembly 152 and 154. In some configurations, the leg cuffs may not overlap the absorbent assembly. It is to be appreciated that the leg cuffs may be formed in various ways, such as for example, by folding portions of the chassis 102 laterally inward, i.e., toward the longitudinal axis 124, to form both the respective leg cuffs and the side edges 128 and 130 of the chassis 102. In another example, the leg cuffs may be formed by attaching an additional layer or layers to the chassis at or adjacent to each of the respective side edges and of the chassis. Each of the leg cuffs may be joined to the interior surface 132 of the chassis and/or the absorbent assembly in leg cuff attachment zones in the front waist region 116 and in leg cuff attachment zones in the back waist region 118. The leg cuffs may extend to the same longitudinal extent as the absorbent article or alternatively the leg cuffs may have a longitudinal extent that is less than the absorbent article.

The elasticized waistband 158 may provide improved fit and containment and may be a portion or zone of the diaper 100 that may elastically expand and contract to dynamically fit a wearer's waist. It is to be appreciated that the elasticized waistband 158 may be located in various positions relative to various diaper components. For example, the elasticized waistband 158 may be positioned longitudinally inwardly from the waist edges 120, 122 of the diaper and/or toward the lateral edges 148, 150 of the absorbent core 142. In some configurations, the elasticized waistband 158 may be positioned with a lateral edge that is coterminous with the waist edges 120, 122. In some configurations, the elasticized waistband 158 may be positioned such that laterally opposing end regions of the waistband 158 are located laterally inward from the leg cuffs 156. In some configurations, the elasticized waistband 158 may be positioned such that laterally opposing end regions of the waistband 158 overlap the leg cuffs 156. In some configurations, the elasticized waistband 158 may be positioned on the wearer facing surface 132 of the topsheet 138. In some configurations, the waistband 158 may be positioned on the wearer facing surfaces 132 of the topsheet 138 and the leg cuffs 156. In some configurations, the waistband 158 may be positioned on the wearer facing surfaces 132 of the topsheet 138 and laterally opposing end regions of the waistband 158 may be positioned between the leg cuffs 156 and the topsheet 138. In some configurations, the elasticized waistband 158 may be positioned between the garment facing surface 132 of the topsheet 138 and the wearer facing surface 132 of the backsheet 136. And in some configurations, the elasticized waistband 158 may be positioned on the garment facing surface 134 of the backsheet 136. The diaper 100 may also include more than one elasticized waistband 158, for example, having one waistband 158 positioned in the back waist region 118 and one waistband 158 positioned in the front wait region 116, although other embodiments may be constructed with a single elasticized waistband 158. The elasticized waistband 158 may be constructed in a number of different configurations including those described in U.S. Pat. Nos. 4,515,595 and 5,151,092.

Taped diapers may be manufactured and provided to consumers in a configuration wherein the front waist region and the back waist region are not fastened, pre-fastened, or connected to each other as packaged, prior to being applied to the wearer. For example, the taped diaper 100 may be folded about a lateral centerline with the interior surface 132 of the first waist region 116 in surface to surface contact with the interior surface 132 of the second waist region 118 without fastening or joining the waist regions together. The rear side panels 104 and 106 and/or the front side panels 108 and 110 may also be folded laterally inward toward the inner surfaces 132 of the waist regions 116 and 118.

The diaper 100 may also include various configurations of fastening elements to enable fastening of the front waist region 116 and the back waist region 118 together to form a closed waist circumference and leg openings once the diaper is positioned on a wearer. For example, as shown in FIGS. 1A and 1B, the diaper 100 may include first and second fastening members 162, 164, also referred to as tabs, connected with the first and second rear side panels 104, 106, respectively. The diaper may also include first and second front side panels 108, 110, that may or may not include fastening members.

With continued reference to FIGS. 1A and 1B, each side panel 104, 106 and/or fastening member 162 and 164 may form a portion of or may be permanently bonded, adhered or otherwise joined directly or indirectly to the chassis 102 laterally inward from the side edge 128 and 130, in one of the front waist region 116 or the back waist region 118. Alternatively, the fastening members 162, 164 may form a portion of or may be permanently bonded, adhered or otherwise joined directly or indirectly to the first and second rear panels 104, 106 at or adjacent the distal edge of the panel and/or the first and second front side panels 108 and 110 at or adjacent the distal edge of the side panel. It is to be appreciated that the fastening members and/or side panels may be assembled in various ways, such as disclosed for example, in U.S. Pat. No. 7,371,302. The fastening members 162, 164 and/or side panels 104, 106, 108, 110 may also be permanently bonded or joined at or adjacent the side edges 128 and 130 of the chassis 102 in various ways, such as for example, by adhesive bonds, sonic bonds, pressure bonds, thermal bonds or combinations thereof, such as disclosed for example, U.S. Pat. No. 5,702,551, which is incorporated by reference herein.

Referring now to FIG. 1B, the first fastening member 162 and/or the second fastening member 164 may include various types of releasably engageable fasteners. The first and second fastening members 162 and/or 164 may also include various types of refastenable fastening structures. For example, the first and second fastening members 162 and 164 may include mechanical fasteners, 166, in the form of hook and loop fasteners, hook and hook fasteners, macrofasteners, buttons, snaps, tab and slot fasteners, tape fasteners, adhesive fasteners, cohesive fasteners, magnetic fasteners, hermaphroditic fasteners, and the like. Some examples of fastening systems and/or fastening members 162, 164 are discussed in U.S. Pat. Nos. 3,848,594; 4,662,875; 4,846,815; 4,894,060; 4,946,527; 5,151,092; 5,221,274; 6,251,097; 6,669,618; 6,432,098; and U.S. Patent Publication Nos. 2007/0078427 A1 and 2007/0093769 A1, which are all incorporated by reference herein.

As previously mentioned, the fastening members 162 and 164 may be constructed from various materials and may be constructed as a laminate structure. The fastening members 162 and 164 may also be adapted to releasably and/or refastenably engage or connect with another portion of the diaper 100. For example, as shown in FIG. 1A, the diaper 100 may include a connection zone 168, sometimes referred to as a landing zone, in the first waist region 116. As such, when the taped diaper 100 is placed on a wearer, the fastening members 162 and 164 may be pulled around the waist of the wearer and connected with the connection zone 168 in the first waist region 116 to form a closed waist circumference and a pair of laterally opposing leg openings. It is to be appreciated that the connection zone may be constructed from a separate substrate that is connected with the chassis 102 of the taped diaper. In some embodiments, the connection zone may be integrally formed as part of the backsheet 136 of the diaper 100 or may be formed as part of the first and second front panels 108, 110, such as described in U.S. Pat. Nos. 5,735,840 and 5,928,212, which are both incorporated by reference herein.

As previously mentioned, absorbent articles may be assembled with various components that may constructed with the substrates described herein. Thus, in the context of the previous discussion, the apparatuses and methods herein may be used to bond discrete elastic parts under tension to an advancing carrier substrate during the assembly of an absorbent article 100. For example, the apparatuses and methods herein may be utilized to bond elastic parts that may be configured as waistbands 158 to carrier substrates that may be configured as topsheets 138 or backsheets 136 during the manufacture of absorbent articles 100. It is to be appreciated that the systems and methods disclosed herein are applicable to work with various types of converting processes and/or machines. For example, FIG. 2 shows a schematic representation of a converting process including an apparatus or system 300 that bonds discrete elastic parts 200 with an advancing carrier substrate 202 to form a laminate 204.

As shown in FIGS. 2 and 3, the carrier substrate 202 may advance in a machine direction MD at a first speed S1. The carrier substrate comprises a first longitudinal edge 206 and a second longitudinal edge 208 separated from the first longitudinal edge 206 in a cross direction CD to define a width Wes. The carrier substrate 202 also includes a first surface 210 and an opposing second surface 212. As discussed in more detail below, discrete elastic parts 200 are bonded with the first surface 210 of the carrier substrate 202.

In the context of components of absorbent articles 100 discussed above and assembly processes thereof, the elastic parts 200 may be configured as waistbands 158 and the carrier substrate 202 may be configured as a continuous topsheet 138, backsheet 136, or continuous laminate of a combined topsheet 138 and backsheet 136. As such, the first surface 210 of the carrier substrate 202 may correspond with the wearer facing surface 132 or the garment facing surface 134 of the topsheet 138 or backsheet 136. In some configurations, the elastic part 200 may be bonded between a topsheet 138 and a backsheet 136. For example, the elastic part 200 may be bonded with the wearer facing surface 132 of the backsheet 136, which is subsequently bonded with a topsheet 138. In another example, the elastic part 200 may be bonded with the garment facing surface 134 of the topsheet 138, which is subsequently bonded with a backsheet 136. In yet another example, the elastic part 200 may be bonded with the garment facing surface 134 of the backsheet 136, wherein the wearer facing surface 132 of the backsheet 136 may have been previously bonded with a topsheet 138 or may be subsequently bonded with a topsheet 138. In another example, the elastic part 200 may be bonded with the wear facing surface 132 of the topsheet 136, wherein the garment facing surface 134 of the topsheet 138 may have been previously bonded with a backsheet 136 or may be subsequently bonded with a backsheet 136.

As shown in FIG. 3A, the carrier substrate 202 may also include leg cuffs 156 positioned on the first surface 210 adjacent the first longitudinal edge 206 and the second longitudinal edge 208. As such, portions of the discrete elastic parts 200 may also be bonded with the leg cuffs 156. In some configurations, the discrete elastic parts 200 may be bonded with the carrier substrate 202 and leg cuffs 156 may subsequently be bonded with the carrier substrate 202. The leg cuffs 156 may be positioned relative the elastic part 200 such that the leg cuffs 156 may or may not partially cover or overlap opposing end portions of the elastic part 200. In some configurations, the leg cuffs 156 may be sandwiched between the elastic parts 200 and the carrier substrate 202. And in some configurations, the elastic parts 200 may be sandwiched between the leg cuffs 156 and the carrier substrate 202.

Referring now to FIGS. 2 and 4, a continuous elastic substrate 200a advanced at a second speed S2 in a machine direction MD, wherein the second speed S2 is less than the first speed S1. The continuous elastic substrate 200a comprises a first longitudinal edge 214 and a second longitudinal edge 216 separated from the first longitudinal edge 214 in the cross direction CD to define a width $W_{ES}$. The continuous elastic substrate 200a also includes a first surface 218 and an opposing second surface 220. The continuous elastic substrate 200a is stretchable in at least one direction and is oriented such that the continuous elastic substrate 200a is stretchable in the machine direction MD. As such, the continuous elastic substrate may be unstretched in the machine direction MD. In some configurations, the continuous elastic substrate 200a may be partially stretched in the machine direction MD.

With continued reference to FIGS. 2, 4, and 5, the system 300 may include an adhesive applicator device 302 that deposits adhesive 222 onto the second surface 220 of the continuous elastic substrate 200a. It is to be appreciated that the adhesive applicator device 302 may be configured in various ways, such as for example, as a spray nozzle and/or a slot coating device. In some configurations, the adhesive applicator device 302 may be configured in accordance with the apparatuses and/or methods disclosed in U.S. Pat. Nos. 8,186,296; 9,265,672; 9,248,054; and 9,295,590 and U.S. Patent Publication No. 2014/0148773 A1, which are all incorporated by reference herein.

It is to be appreciated that the adhesive 222 may be applied to the continuous elastic substrate 200a to define regions 224 of adhesive 222 on the second surface 220 having various shapes and sizes relative to the continuous elastic substrate 200a. For example, as shown in FIG. 5, the adhesive 222 may be applied to the second surface 220 of the continuous elastic substrate 200a to define regions 224 of adhesive 222 that are spaced part in the machine direction MD and each extending continuously in the cross direction CD between the first and second longitudinal edges 214, 216. The adhesive 222 may extend in the cross direction CD to define a width $W_{ADH}$. In some configurations, the width $W_{ADH}$ of adhesive 222 may be equal to the width $W_{ES}$ of the continuous elastic substrate 200a, and in other configurations, the width $W_{ADH}$ may be less than the width $W_{ES}$ of the continuous elastic substrate 200a (e.g., FIG. 5B).

As shown in FIGS. 2, 5, and 6, the continuous elastic substrate 200a may advance in the machine direction MD from the adhesive applicator device 302 to a cutting device 304 that cuts and separates discrete elastic parts 200 from the continuous elastic substrate 200a. As such, the discrete elastic parts 200 each include a leading edge 230 and a trailing edge 232 and defines a first length $L_{EP1}$ in the machine direction MD extending from the leading edge 230 to the trailing edge 232. The elastic part 200 also includes first and second longitudinal edges 214, 216 that correspond with the longitudinal edges 214, 216 of the continuous elastic substrate 200a extending between the leading and trailing edges 230, 232. In addition, the elastic part 200 includes first and second surfaces 218, 220 that correspond with the first and second surfaces 218, 220 of the continuous elastic substrate 200a.

As shown in FIG. 6, the discrete elastic part 200 also includes a top region 235 adjacent the trailing edge 232 and a bottom region 237 adjacent the leading edge 230, wherein the bottom region 237 is separated from the top region 235 in the machine direction MD by a central region 238. As discussed above, adhesive 222 may be applied to the second surface 220 of the continuous elastic substrate 200a. As such, the discrete elastic part 200 may include a zone 240 of adhesive 222 on the second surface 220. It is to be appreciated that the zone 240 of adhesive 222 may define various sizes and shapes relative to the elastic part 200. For example, as shown in FIG. 6, the zone 240 of adhesive may extend in the machine direction MD for less than the entire length $L_{EP1}$ of the discrete elastic part 200. In some configurations, the zone 240 of adhesive 222 may be positioned only on the central region 238 of the discrete elastic part 200 such that the top region 235 and the bottom region 237 of the second surface 220 of the discrete elastic part 200 may not include any adhesive 222.

As shown in FIGS. 2 and 9, the cutting device 304 may include a knife roll 306 positioned adjacent an anvil roll 308 to define a nip 310 therebetween. The knife roll 306 may include an outer circumferential surface 312 and one or more blades 314 adapted to rotate about an axis 316 in a first direction Dir1. The anvil roll 308 may include an outer circumferential surface 318 adapted to rotate about an axis 320 in a second direction Dir2 opposite the first direction Dir1 such that the outer circumferential surface 318 advances at a third speed S3, wherein the third speed S3 is greater than the second speed S2. With continued reference to FIG. 2, as the continuous elastic substrate 200a advances through the nip 310 between the knife roll 306 and the anvil roll 308, the blade 314 operates to cut the discrete elastic part 200 from the continuous elastic substrate 200a. Because the outer circumferential surface 318 of the anvil roll 308 advances at the third speed S3, the cut discrete elastic part 200 may then accelerate from the second speed S2 to the third speed S3 on the outer circumferential surface 318 of the anvil roll 308. In some configurations, the third speed S3 may be greater than the second speed S2 and as such, the discrete elastic part 200 is accelerated on the anvil roll 308 such that the first length $L_{EP1}$ of the discrete elastic part 200 (e.g., measured along a circumference of the anvil roll 308) increases to a second length $L_{EP2}$ by the anvil roll 308. In an example embodiment, the first length is about 150 millimeters (mm) or in a range from about 120 mm to about 180 mm and the second length is about 200 mm or in a range from about 160 mm to about 240 mm. FIG. 2 shows one embodiment, where the first length $L_{EP1}$ of the discrete elastic part 200 (upstream of the anvil roll 308) increased to the second length $L_{EP2}$ due to acceleration experienced by the discrete elastic part 200 based on the increase in speed from the second speed S2 to the third speed S3 of the anvil roll 308. In an embodiment, FIG. 6 depicts the discrete elastic part 200 with the first length $L_{EP1}$ in the machine direction upstream of the anvil roll 308 and FIG. 10 depicts the discrete elastic part 200 with the stretched second length $L_{EP2}$ in the machine direction MD after experiencing the force due to acceleration on the anvil roll 308. In some embodiments, a controller of the system 300 is provided and determines a value of the third speed S3 (e.g., greater than the second speed S2) such that the length of the discrete elastic part 200 is extended by a desired amount (e.g., from the first length $L_{EP1}$ to the second length $L_{EP2}$) which may be input by a user of the system. One of ordinary skill in the art would recognize how to calculate values of the second speed S2 and third speed S3 in order to achieve this extension in length.

Additionally, in some embodiments, in addition to stretching the discrete elastic part 200 in the MD direction, the anvil roll 308 may introduce a spacing between adjacent discrete elastic parts 200 that are received on the anvil roll 308. In these embodiments, the outer surface of the anvil roll 308 may be configured with a coefficient of kinetic friction such that the outer surface slips over each discrete elastic part 200 for a controlled distance before the outer surface grips the discrete elastic part 200 and accelerates the discrete elastic part 200. In these embodiments, this limited slippage between the outer surface of the anvil roll 308 and the discrete elastic part 200 introduces a desired spacing between adjacent discrete elastic parts 200 in the machine direction MD prior to the anvil roll 308 expanding the length of each discrete elastic part 200.

In other configurations, the third speed S3 may be equal to the first speed S1 of the advancing carrier substrate 202. In some configurations, the third speed S3 may be less than or greater than the first speed S1 of the advancing carrier substrate 202, and as such, the discrete elastic part may be accelerated or decelerated downstream of the anvil roll 308 from the third speed S3 to the first speed S1 before being combined with the carrier substrate 202. Because the first speed S1 of the carrier substrate is greater than the second speed S2, the discrete elastic parts 200 are accelerated from the second speed S2 to the first speed S1 before bonding with the carrier substrate 202. By accelerating discrete elastic parts 200 from the second speed S2 to the first speed S1, trailing edges 232 (or leading edges 230) of consecutively cut discrete elastic parts 200 may be separated from each other in the machine direction MD by a pitch distance PD, such as shown in FIG. 12, which may correspond with the pitch length PL described above with reference to FIGS. 1A and 1B. The anvil roll 308 may also be configured to apply vacuum pressure to the discrete elastic parts 200 to help hold the discrete elastic parts 200 on the outer circumferential surface 318 as the anvil roll 308 rotates and stretches the length of the discrete elastic parts 200 from the first length $L_{EP1}$ to the second length $L_{EP2}$ along the machine direction MD.

It is to be appreciated that the cutting device 304 may be configured in various ways. For example, in some configurations, the blade 314 may be configured such that resulting cut lines and corresponding leading edges 230 and trailing edges 232 of the discrete elastic parts 200 may be straight and/or curved. The cutting device 304 may also be adapted to cut the discrete elastic parts 200 such that material along the cut line adjacent leading edges 230 and trailing edges 232 is fused and/or pressure bonded together. It is also to be appreciated that the positions of the knife roll 306 and anvil roll 308 may be opposite to that which is illustrated in FIG. 2, and as such, the discrete elastic parts 200 may remain on the outer circumferential surface 312 of the knife roll 306 as opposed to the anvil roll 308. It is also to be appreciated that the cutting device 304 may be configured to convey and/or cut the discrete elastic parts 200 in different ways. For example, the cutting device 304 may be adapted to advance the continuous elastic substrate 200a and/or the discrete elastic parts 200 on a conveyor belt. In another example, the cutting device 304 may include a laser adapted to cut the discrete elastic parts 200 from the continuous elastic substrate 200a. It is also to be appreciated that one or more components of the cutting device 304 may be configured to operate at constant and/or variable speeds. For example, the knife roll 306 and/or the anvil roll 308 may be connected with various types of motors, such as servo motors for example, that may rotate the knife roll 306 and/or the anvil roll 308 at constant and/or variable angular velocities.

With reference to FIG. 2, the apparatus 300 may include a rotatable transfer device 322 that transfers the discrete elastic parts 200 from the cutting device 304 to a bonding device 324, which in turn, combines the elastic parts 200 with the carrier substrate 202. The transfer device 322 may also be configured to rotate the discrete elastic parts 200 by an angle (e.g., 90 degrees) so that the second length $L_{EP2}$ is aligned in the cross direction CD (FIG. 10A). As such, the transfer device 322 may be configured as a rotational mechanism 326, such as shown in FIGS. 2 and 9. With continued reference to FIGS. 2 and 9, the transfer device 322 may be positioned adjacent the anvil roll 308 to define a nip 328 therebetween. In some configurations, the anvil roll 308 may be configured to apply positive air pressure, sometimes referred to as blow-off air, to the discrete elastic parts 200 adjacent the nip 328 to help remove the discrete elastic parts 200 from the anvil roll 308 during transfer to the transfer device 322. As discussed in more detail below, the discrete elastic parts 200 are received from the anvil roll 308 and the rotational mechanism 326 operates to rotate discrete elastic parts 200 by the angle (e.g., 90 degrees) so that the second length $L_{EP2}$ is aligned in the cross direction CD. More specifically, as shown in FIGS. 6 and 10A, the rotational mechanism 326 is configured to rotate a longitudinal axis 239 of the discrete elastic part 200 from being aligned with the machine direction MD (FIG. 6) to the cross direction CD (FIG. 10A). The stretched discrete elastic parts 200 are then advanced from the rotational mechanism 326 onto a rotating component of the bonding device 324, which in turn, bonds the stretched discrete elastic parts 200 onto the carrier substrate 202.

As shown in FIGS. 2 and 9, in some embodiments the rotational mechanism 326 includes a plurality of axles 374 that are interconnected and collectively rotate about a rotational axis 321 (e.g., in the plane of FIG. 2). In one embodiment, the axles 374 rotate about the rotational axis 321 in a third direction Dir3 that is opposite to the second direction Dir2 of the anvil roll 308. In another embodiment, the axles 374 rotate at a speed that is about equal to the third speed S3 of the anvil roll 308. Additionally, each axle 374 is pivotable about a second rotational axis defined by a longitudinal axis of each respective axle 374 such that each axle 374 pivots in a rotational direction 372 (FIG. 2). In an embodiment, a plate 376 is provided at an end of each axle 374. In some embodiments, the plate 376 is dimensioned based on one or more dimensions of the discrete elastic part 200 after being stretched in the MD direction (FIG. 10). In one example embodiment, the plate 376 has a length that is based on the length $L_{EP2}$ of the discrete elastic part 200 in the machine direction MD and a width that is based on the width w of the discrete elastic part 200 in the cross direction CD. Additionally, in some embodiments the plate 376 is recessed in a central region where it includes a recess 377 (FIG. 9) that is sized based on dimensions of the zone 240 of adhesive 222 on the discrete elastic part 200. The inventors of the present invention recognized that providing this recess 377 in each plate 376 advantageously reduces the instance of the adhesive 222 making contact with the components of the system 300 including the plate 376. FIG. 9A depicts a cross-sectional side view of the discrete elastic part 200 positioned within the recess 377 of the plate 376. As shown in FIG. 9A the discrete elastic part 200 is positioned with the zone 240 of adhesive 222 facing downward in the recess 377. The plate 376 includes a pair of vacuum surfaces 379, 381 at opposite ends of the plate 376. One or more dimensions of the plate 376 and vacuum surfaces 379, 381 are selected such that when the discrete elastic part 200 is positioned facing downward in the recess 377, the top and bottom regions 235, 237 (which do not have adhesive 222) engage the vacuum surfaces 379, 381 whereas the zone 240 of adhesive in the central region 238 does not make contact with the plate 376. It is to be appreciated that this structural configuration may help ensure that adhesive 222 does not contact the plates 376 and thus keeps the adhesive 222 from contacting the components of the system 300.

As with the anvil roll 308, in some embodiments the rotational mechanism 326 may also be configured to apply vacuum pressure to the discrete elastic parts 200 to help hold the discrete elastic parts 200 on each plate 376 as each plate 376 rotates from the anvil roll 308 to the bonding device 324. Additionally, in some embodiments the anvil roll 308 releases the vacuum pressure on each discrete part 200 in the vicinity of the nip 328 and the plate 376 adj acent the nip 328 applies the vacuum pressure so to enable the transfer of the discrete elastic part 200 from the anvil roll 308 to the plate 376. Similarly, in some embodiments the plate 376 releases the vacuum pressure on each discrete elastic part 200 in the vicinity of the nip 338 and the roll 340 of the bonding device 324 applies vacuum pressure so to enable transfer of the discrete elastic part 200 (now aligned in the cross direction CD) to the bonding device 324.

In an embodiment, the rotational mechanism 326 is configured such that plates 376 rotating in the vicinity of the anvil roll 308 are oriented with a long dimension of the plate 376 in the machine direction MD (FIG. 9) in order to accept discrete elastic parts 200 from the anvil roll 308 (where the length $L_{EP2}$ is oriented in the machine direction MD). Additionally, in an embodiment, the rotational mechanism 326 is configured such that the plates 376 rotating in the vicinity of the bonding device 324 are oriented with the long dimension of the plate 376 in the cross direction CD (FIG. 9) in order to orient the discrete elastic part 200 in the cross direction CD before providing the discrete elastic part 200 to the bonding device 324. Thus, in these embodiments, the rotational mechanism 326 is configured such that the plates 376 are oriented towards the machine direction MD near the nip 328 and towards the cross direction CD near the nip 338. Although the rotational mechanism 326 is depicted in FIG. 2, any other means or structure could be utilized in the system 300 that is capable of rotating the discrete elastic part 200 from being oriented in the machine direction MD from the anvil roll 308 to the cross direction CD at the bonding device 324.

Although FIG. 2 describes one embodiment of a rotational mechanism 326 used as the transfer device 322, other embodiments of different rotational mechanisms can be used. In one embodiment, FIG. 2B depicts a system 300' that is similar to the system 300 of FIG. 2 with the exception of the features discussed herein. The system 300' includes a rotational mechanism 326' of the transfer device 322'. Unlike the system 300 of FIG. 2, where the anvil roll 308 introduces spacing between adjacent discrete elastic parts 200, the anvil roll 308 of the system 300' of FIG. 2B does not introduce spacings between adjacent discrete elastic parts 200. Thus, in the embodiment of FIG. 2B, the rotational mechanism 326' not only rotates the discrete elastic part 200 to be aligned in the cross direction CD, but further introduces spacing between adjacent discrete elastic parts 200 when delivered to the bonding device 324. As shown in FIG. 2B, the axles 374 have a smaller rotational velocity adjacent to the anvil roll 308 and a larger rotational velocity adjacent to the bonding device 324. Consequently, as shown in FIG. 2B, the axles 374 are closely spaced adjacent to the anvil roll 308 and further apart adjacent to the bonding device 324. This variation in the angular velocity of the axles 374 introduces a spacing between adjacent discrete elastic parts 200 that are delivered to the bonding device 324. Additionally, as with the rotational mechanism 326 of FIG. 2, the rotational mechanism 326' rotates the discrete elastic parts 200 with the long dimension in the cross direction CD upon delivery to the bonding device 324. As such, it is to be appreciated that the rotational mechanism 326' may introduce spacing between adjacent discrete elastic parts 200, in the event that the anvil roll 308 does not or cannot introduce this spacing.

Although FIGS. 2 and 2A describe embodiments of a rotational mechanism 326, 326' used as the transfer device 322, 322', in other embodiments different rotational mechanisms can be used. In one embodiment, FIG. 2C depicts a system 300" that is similar to the system 300 of FIG. 2 with the exception of the features discussed herein. The system 300" includes a rotational mechanism 326" of the transfer device 322". Unlike the system 300 of FIG. 2, where the anvil roll 308 expands the length of the discrete elastic part 200 from the first length $L_{EP1}$ (FIG. 6) to the second length $L_{EP2}$ (FIG. 10), the anvil roll 308 of the system 300" of FIG. 2C does not expand the length of the discrete elastic part 200. Thus, in the embodiment of FIG. 2C, the rotational mechanism 326" not only rotates the discrete elastic part 200 to be aligned in the cross direction CD, but further expands the length of the discrete elastic part 200 before being delivered to the bonding device 324. As shown in FIG. 2C, each discrete elastic part 200 is secured between two plates 376 that span between two adjacent axles 374. In this embodiment, the leading edge 230 of the discrete elastic part 200 engages the plate 376 of a first axle 374 and the trailing edge 232 of the same elastic part 200 engages the plate 376 of a second axle 374 that is adjacent the first axle 374. In these embodiments, the plates 376 of the adjacent axles 374 apply vacuum pressure to secure the discrete elastic part 200 to both plates 376. Additionally, in these embodiments, as the pair of axles 374 rotate in the direction 372 (FIG. 2C) to rotate the elastic part 200 in the CD direction, a spacing between the adjacent plates 376 simultaneously increases, which causes the length of the discrete elastic part 200 to expand as the discrete elastic part 200 is rotated to the cross direction CD. This increase in separation between the adjacent plates 376 expands the length of the discrete elastic part 200 in a similar extent as previously discussed with the anvil roll 308 (e.g., from the first length $L_{EP1}$ (FIG. 6) to the second length $L_{EP2}$ (FIG. 10)). Additionally, as with the rotational mechanism 326 of FIG. 2, the rotational mechanism 326" rotates the discrete elastic parts 200 with the long dimension in the cross direction CD upon delivery to the bonding device 324. As such, it is to be appreciated that the rotational mechanism 326" may expand the length of the discrete elastic parts 200, in the event that the anvil roll 308 does not or cannot expand this length.

As previously discussed with reference to FIG. 6, the elastic part 200 may include a zone 240 of adhesive 222 that is positioned only on the central region 238 of the discrete elastic part 200 and wherein the top region 235 and the bottom region 237 of the second surface 220 of the discrete elastic part 200 may not include any adhesive 222. As shown in FIGS. 2, 9 and 9A, once transferred to the transfer device 322, the elastic parts 200 may be oriented such that the first surface 218 may be facing radially outward, and the second surface 220 and the zone 240 of adhesive 222 may be facing radially inward. As such, the arrangement of plates 376 of the rotational mechanism 326 provide the ability to rotatably convey the elastic parts 200 from the cutting device 304 to the bonding device 324 with a zone 240 of adhesive 222 that faces radially inward without having to contact the adhesive 222 with the plates 376. Specifically, the plates 376 include the recess 376 in a center region of the plate 376 that is sized based on dimensions of the zone 240 and thus the adhesive 222 does not make contact the plate 376, as previously discussed with respect to FIG. 9A.

It is to be appreciated that the transfer device 322 may be configured in various ways to help ensure a relatively smooth and consistent transfer of the discrete elastic parts 200 from the cutting device 304 to the transfer device 322 as well as a relatively smooth and consistent transfer of the discrete elastic parts 200 from the transfer device 322 to the bonding device 324.

As discussed herein, materials utilized in the make up of the continuous elastic substrate 200a and elastic parts 200 may be subjected to multiple process operations, such as stretching, cutting, bonding, and the like. In some configurations, the continuous elastic substrate 200a and elastic parts 200 may be configured to have desired machine direction MD and/or cross direction CD properties for particular process transformations. For example, when cutting discrete elastic parts 200 from the continuous elastic substrate 200a, if materials stretch or deform due to web tension, the cross direction CD length of the elastic part may vary. In another example, stretching the elastic part 200 in the machine direction MD, if materials are not configured to have proper levels of forces and available strain in the machine direction MD, the elastic part 200 may not stretch correctly and may result in operational and/or product failures. As such, materials used for such processes may be configured with relatively balanced machine direction MD and cross directional CD properties. In some instances, such balancing may be defined with property ratios. For example, a ratio may be defined by elongation at peak, according to the Tensile Test Method herein. In some configurations, material may have an (elongation at peak in MD)/(elongation at peak in CD) ratio of 1.5 or higher; 2 or higher; 2.5 or higher; and 3 or higher. Such a ratio may indicate that elastic material (collected from the center of the elastic part 200 and the continuous elastic substrate 200a for both CD and MD measurements) has an elongation at peak in machine direction MD that is 1.5 times higher than material elongation at peak in the cross direction CD. In some configurations, the material CD/MD tensile force ratio at 5% elongation may be at least 1.5 times, indicating that when material collected from the center of the elastic part 200 and the continuous elastic substrate 200a for both CD and MD measurements is pulled in cross direction CD exhibits a 1.5 times higher force, 2 times higher force, 3 times higher force, or 4 (or greater) times higher force at 5% elongation than a sample collected from the center of the elastic part 200 and the continuous elastic substrate 200a pulled in machine direction MD to 5% elongation, according to the Tensile Test Method herein.

It is to be appreciated that various drives may be used to control the rotation of the plates 376 of the rotational mechanism 326. In some embodiments, the rotation of the axles 374 and plates 376 about the rotational axis 321 is controlled and/or the rotation of each axle 374 (about its own longitudinal axis) is controlled. For example, the axles 374 and plates 376 of the rotational mechanism 326 may be driven by one or more motors, such as a servo motor. In some configurations, motors may be directly connected with the axles 374 and in some configurations, motors may be indirectly connected with the axles 374, such as through belts, pulleys, and/or gears. The axles 374 may be driven through the use of a common driveshaft. In some configurations, a common jackshaft may be used to drive the axles 374 together with a single motor. In some configurations, drives of components of the cutting device 304 and/or the bonding device 324 and rotational mechanism 326 may be operatively connected, and may be configured with a single motor. In another embodiment, the rotational mechanism 326 may also change the speed at which the discrete elastic part 200 advances in the machine direction MD. It is to be appreciated that various forms of the rotational mechanisms 326, 326', 326" may be used with the methods herein, such as for example, a carrier apparatus as disclosed in U.S. Pat. No. 7,587,966; U.S. Patent No. 9,168,182; U.S. Patent No. 9,737,442; U.S. Patent Publication No. 2021/0267812; and U.S. Patent Publication No. 2022/0008256; the entire contents of each are incorporated by reference herein.

As discussed above, the cut discrete elastic parts 200 accelerate from the second speed S2 to the third speed S3 on the outer circumferential surface 318 of the anvil roll 308, which causes the discrete elastic part 200 to stretch in the machine direction MD from the first length $L_{EP1}$ to the second length $L_{EP2}$. In some configurations, the third speed S3 may be less than or greater than the first speed S1 of the advancing carrier substrate 202. Thus, the transfer device 322 may be configured to rotate at a variable angular velocity to accelerate or decelerate the discrete elastic parts 200 to the first speed S1. For example, if the third speed S3 is less than the first speed S1, the transfer device 322 may be configured to receive the discrete elastic part 200 from the anvil roll 308 while the plates 376 are moving through the nip 328 at the third speed S3. The angular velocity of the plates 376 may then be changed to accelerate the discrete elastic part 200 to the first speed S1 before transferring the discrete elastic part 200 to the bonding device 324. In another example, if the third speed S3 is greater than the first speed S1, the angular velocity of the plates 376 may be changed to decelerate the discrete elastic part 200 to the first speed S1 before transferring the discrete elastic part 200 to the bonding device 324. In situations where the third speed S3 is equal to the first speed S1, the plates 376 may rotate at a constant angular velocity. It is to be appreciated that the rotational mechanism 326 may be configured in various ways to accommodate a need to rotate at variable angular velocities, such as, for example, disclosed in European Patent Publication No. EP 2260813 B1, which is incorporated by reference herein. The ability to rotate at the rotational mechanism 326 at variable angular velocities may help reduce the need to replace components of the apparatus 300 when assembling absorbent articles 100 of smaller or larger sizes, which in turn, may require a reduction or increase in the pitch distances between consecutively cut discrete elastic parts 200. However, regardless of how the angular velocity of the plates 376 is varied, the rotational speed of the axles 374 (about their respective axes) is adjusted such that the plate 376 rotates the discrete elastic part 200 from the longitudinal axis 239 being aligned in the machine direction MD (FIG. 10) when the elastic part 200 is received from the anvil roll 308 to being aligned in the cross direction CD (FIG. 10A) when the elastic part 200 is transferred to the bonding device 324.

As previously mentioned, the transfer device 322 may be configured to transfer the discrete elastic parts 200 from the cutting device 304 to a bonding device 324. As shown in FIGS. 2 and 9, the bonding device 324 may be positioned adjacent the plates 376 of the rotational mechanism 326 to define a nip 338 therebetween. In some configurations, the plates 376 may be configured to apply positive air pressure, sometimes referred to as blow-off air, to the discrete elastic part 200 adjacent the nip 338 to help remove the discrete elastic parts 200 from the plates 376 during transfer to the bonding device 324. As discussed in more detail below, the discrete elastic parts 200 are received from the rotational mechanism 326 with the central regions 238 stretched in the cross direction CD (FIG. 10A), and the bonding device 324 transfers and bonds the discrete elastic parts 200 in the stretched state to the advancing carrier substrate 202.

It is to be appreciated that the bonding device 324 may be configured in various ways. For example, as shown in FIGS. 2 and 9, the bonding device 324 may be configured with a pattern roll 340 and a pressing surface 342 adjacent the pattern roll 340 to define a nip 344 therebetween. The pattern roll 340 includes an outer circumferential surface 346 and rotates about an axis of rotation 348, wherein the pattern roll 340 may rotate in a fourth direction Dir4 that is opposite the third direction Dir3. In addition, pattern roll 340 may rotate such that the outer circumferential surface 346 advances at or about the first speed S1. During operation, discrete elastic parts 200 in a stretched state are transferred from the plates 376 to the outer circumferential surface 346 of the pattern roll 340. The pattern roll 340 rotates to advance the stretched elastic parts 200 between the outer circumferential surface 346 of the pattern roll and the advancing carrier substrate 202. In particular, the first surface 218 of the discrete elastic part 200 may be positioned in a facing relationship with and in direct contact with the outer circumferential surface 346 of the pattern roll 340. As such, the zone 240 of adhesive 222 and the second surface of the discrete elastic part 200 may be facing radially outward from the rotation axis 348. The carrier substrate 202 advances to the pattern roll 340 such that the first surface 210 of the carrier substrate 202 is in direct contact with and in a facing relationship with the outer circumferential surface 346 of the pattern roll 340. As the pattern roll 340 rotates, the second surface 220 of the discrete elastic part 200 is positioned in direct contact with and in a facing relationship with the first surface 210 of the carrier substrate 200. The combined discrete elastic part 200 and the carrier substrate 202 advance through the nip 344 between the pattern roll 340 and the pressing surface 342 to mechanically bond the discrete elastic part 200 and the carrier substrate 202 together.

For example, as shown in FIG. 2, the bonding device 324 may be configured as a mechanical bonding device that includes an anvil roll 350. The anvil roll 350 may include an outer circumferential surface 352 and rotates about an axis of rotation 354, wherein the anvil roll 350 may rotate in a fifth direction Dir5 that is opposite the fourth direction Dir4. The outer circumferential surface 352 of the anvil roll 350 may define the pressing surface 342 operating in conjunction with the pattern roll 340. As shown in FIGS. 11 and 11A, the outer circumferential surface 346 of the pattern roll 340 may also comprise one or more bonding surfaces 356 defined by bonding elements 358 extending radially outward. As the pattern roll 340 rotates, the discrete elastic parts 200 and the carrier substrate 202 are advanced between the bonding surfaces 356 and the pressing surface 342 to mechanically bond or weld the elastic part 200 and the carrier substrate 202 together to create bonds 242 between the elastic part 200 and the carrier substrate 202. Heat and/or pressure between the pressing surface 342 and the pattern roll 340 may melt and bond the carrier substrate 202 and the elastic part 200 together in areas supported by the bonding surfaces 356 on the pattern roll 340. As shown in FIG. 12, the mechanical bonds and/or bond regions 242 may have shapes that correspond with and may mirror shapes of the bonding surfaces 356.

Thus, as the laminate 204 advances through the nip 344, the carrier substrate 202 and the discrete elastic part 200 are mechanically bonded or welded together. It is to be appreciated that the bonding device 324 herein may be configured in various ways with various features described herein to bond the discrete elastic parts 200 with the carrier substrate 202. As such, the pattern roll 340 and/or anvil roll 350 may be configured to apply heat and pressure in various ways to perform mechanical bonding, such as for example, the mechanical bonding devices and methods disclosed in in U.S. Pat. Nos. 4,854,984; 6,248,195; 8,778,127; 9,005,392; 9,962,297; and 10,052,237. It is also to be appreciated that the positions of the pattern roll 340 and anvil roll 350 may be opposite to that which is illustrated in FIG. 2, and as such, the discrete elastic parts 200 may be transferred from the transfer device 322 to the outer circumferential surface 352 of the anvil roll 350 as opposed to the pattern roll 340. It is also to be appreciated that one or more components of the bonding device 324 may be configured to operate at constant and/or variable speeds. For example, the pattern roll 340 and/or the anvil roll 350 may be connected with various types of motors, such as servo motors for example, that may rotate the pattern roll 340 and/or the anvil roll 350 at constant and/or variable angular velocities.

In some configurations, the carrier substrate 202 may be partially wrapped around the outer circumferential surface 346 of the pattern roll 340. As such, the bonding device 324 may include one or more rolls that help guide the carrier substrate 202 to and/or from the pattern roll 340. For example, as shown in FIG. 2, the bonding device may include a guide roll 360 that helps to guide the carrier substrate 202 onto the outer circumferential surface 346 of the pattern roll 340 downstream of the nip 338 where the elastic parts 202 are received from the transfer device 322 and upstream of the nip 344 between the pattern roll 340 and the pressing surface 342. The guide roll 360 may also be configured to apply pressure against the carrier substrate 202 and the elastic part 200 to help enhance the bonding of the adhesive 222 of the adhesive zone 240 and the carrier substrate 202.

It is to be appreciated that the bonding device 324 may be configured in various ways, such as with heated or unheated pattern rolls, anvil rolls and/or ultrasonic bonding devices. For example, the bonding device 324 schematically shown in FIG. 2A may include the pattern roll 340 and the pressing surface 342 that comprises an energy transfer surface 362 of an ultrasonic bonding device 364. As such, the bonding device 364 may include a horn 366 and may be configured to impart ultrasonic energy to the combined elastic part 200 and the carrier substrate 202 on the pattern roll 340.

It is to be appreciated that aspects of the ultrasonic bonding device 364 may be configured in various ways, such as for example linear or rotary type configurations, and such as disclosed for example in U.S. Pat. Nos. 3,113,225; 3,562,041; 3,733,238; 5,110,403; 6,036,796; 6,508,641; and 6,645,330. In some configurations, the ultrasonic bonding device 364 may be configured as a linear oscillating type sonotrode, such as for example, available from Herrmann Ultrasonic, Inc. In some configurations, the sonotrode may include a plurality of sonotrodes nested together in the cross direction CD. It is also to be appreciated that rotary horns may also be configured to rotate at constant and/or variable angular velocities.

As discussed above, the pattern roll 340 includes bonding elements 358 that extend radially outward to define bonding surfaces 356. In turn, the bonds and/or bond regions 242 between the discrete elastic part 200 and the carrier substrate 202 may have shapes that correspond with and may mirror shape of the bonding surfaces 356. It is to be appreciated that the pattern roll 340 may have various quantities and/or shapes of bonding surfaces 356 and that such bonding surfaces 356 may be positioned in various locations on the pattern roll 340. For example, as shown in FIGS. 11, 11A, 12, and 13, the bonding elements 358 and bonding surfaces 356 may be positioned to correspond with the first end region 234 and the second end region 236 of the discrete elastic part 200. Thus, the bonding device 340 may operate to mechanically bond the first and second end regions 234, 236 of the elastic part 200 without mechanically bonding the stretched central region 238. In some configurations, the bonding elements 358 and bonding surfaces 356 may be positioned such that mechanical bonds 242 are also applied to bond the central region 238 of the discrete elastic part 200 and the carrier substrate 202 together.

The pattern roll 340 may also be configured to apply vacuum pressure to the discrete elastic parts 200 to help hold the discrete elastic parts 200 on the outer circumferential surface 346 as the pattern roll 340 rotates. The vacuum pressure may also help hold the discrete elastic parts 200 in the stretched state while positioned on the pattern roll 340. In addition, the bonding elements 358 and bonding surfaces 356 may also help grip the elastic parts 200 and help hold the elastic parts 200 in the stretched state. In addition, the pattern roll 340 may be configured such to also apply vacuum pressure through the bonding surfaces 356 of the bonding elements 358. For example, the bonding elements 358 may be configured with through holes that allow vacuum air to be drawn through the bonding surfaces 356. Further, the pattern roll 340 may be configured to interface with the plates 376 of the rotational mechanism 326 to help maintain the stretched state of the discrete elastic part 200 during the transfer to the pattern roll 340 at the nip 338.

As shown in FIG. 2, after the discrete elastic part 200 is bonded with the carrier substrate 202 to create the laminate 204, the laminate 204 may continue to advance in the machine direction MD from the bonding device 324 and may be subjected to additional converting operations, such as cutting, folding, and/or packaging operations. In some configurations, the laminate 204 may define a continuous length of absorbent articles or may be combined with additional substrates and/or components to define a continuous length of absorbent articles. In turn, the continuous length of absorbent articles may be subjected to a final knife cut that separates discrete absorbent articles from the continuous length of absorbent articles. As previously mentioned, the discrete elastic parts 200 may correspond with waistbands 158 on the absorbent articles 100 and the carrier substrate 202 may correspond with a topsheet substrate 138 or backsheet substrate 136. In some configurations, the apparatuses and methods herein may be configured to apply discrete elastic parts 200 as discrete front and/or back waistbands 158. In some configurations, the discrete elastic parts 200 may be applied to the carrier substrate 202, and the discrete elastic parts 200 are subsequently cut during the final knife cut operation into a front waistband 158 positioned in the front waist region 116 and a back waistband 158 positioned in the back waist region 118. It is to be appreciated that such final knife cut operation may be configured to apply straight and/or curved cut lines through the carrier substrate 202 and discrete elastic parts 200. For example, FIG. 12C shows a view of the laminate 204 showing elastic parts 200 and the carrier substrate 202 after being subjected to a final knife cut operation that applies cut lines 231 through the carrier substrate 202 and discrete elastic parts 200. As such, the discrete elastic parts 200 may be cut into a first elastic part 200 b and a second elastic part 200 c. In some configurations, the first elastic part 200 b may correspond with a front waistband 158 positioned in the front waist region 116, and the second elastic part 200 c may correspond with a back waistband 158 positioned in the back waist region 118.

In another example, FIG. 12D shows a view of the laminate 204 showing elastic parts 200 and the carrier substrate 202 after being subjected to a final knife cut operation that applies curved cut lines 231 through the carrier substrate 202 and discrete elastic parts 200. It is to be appreciated that the first elastic part 200 b and the second elastic part 200 c may be cut along lines such that the first elastic part 200 b and the second elastic part 200 c may have shapes that are complimentary or not complimentary with respect to each other. In some configurations, the curved cut line 231 may be adapted to create an umbilical cord notch 233a, such as disclosed in U.S. Pat. No. 8,608,720 and U.S. Patent Publication No. 2017/0246052 A1, both of which are incorporated by reference herein. Thus, it is to be appreciated that such an umbilical cord notch 233a may be formed in both a front waist band 158 and the chassis 102 of a diaper 100. It is also be appreciated that in some configurations, the back waistband 158 may include an extension 233 b that may have a shape that is complimentary with respect to the umbilical cord notch 233a. It is to be further appreciated that in some configurations, the back waistband 158 may include an extension 233 b that may have a shape that is not complimentary with respect to the umbilical cord notch 233a.

It is also to be appreciated that the first elastic part 200 b and the second elastic part 200 c such as shown in FIGS. 12C and 12D may extend in the machine direction MD for the same lengths or for different lengths. As such, it is to be appreciated that a front waistband 158 positioned in the front waist region 116 and a back waistband 158 positioned in the back waist region 118 may extend longitudinally for the same or different distances. In some configurations, front waistbands 158 may define a longitudinal length of 30 mm, and back waistbands may define a longitudinal length of 30 mm. In some configurations, front waistbands 158 may define a longitudinal length of 20 mm, and back waistbands may define a longitudinal length of 40 mm. In some configurations, front waistbands 158 may define a longitudinal length of 10 mm, and back waistbands may define a longitudinal length of 50 mm.

It is also to be appreciated that the carrier substrate 202 may include parts, such as laterally extending side panels for example, attached thereto upstream of the bonding device 324. As such, the system 300 may also include devices, such as rails and/or conveyors, to help guide and control the carrier substrate 202, and specifically such laterally extending features, into the bonding device 324 to help prevent unintentional bonding of such features.

As discussed above, the discrete elastic parts may be combined with the carrier substrate with adhesive and/or mechanical bonds. It is to be appreciated that the adhesive and mechanical bonds may be configured in various ways. For example, as discussed above with reference to FIGS. 2, 4, 5, and 6, the continuous elastic substrate 200a may define a width $W_{ES}$ in the cross direction CD, and the discrete elastic part 200 may also define a first width w in the cross direction CD upstream of the nip 328 between the cutting device 304 and the transfer device 322. And the respective widths $W_{ES}$, w of the continuous elastic substrate 200a and the elastic part 200 may be unstretched widths or partially stretched widths.

As shown in for example FIGS. 4A and 4A1, the continuous elastic substrate 200a may comprise corrugations 244 oriented so as to define corrugation lines 246 extending in the cross direction CD. As discussed above with reference to FIG. 2 and as shown in FIG. 5A, adhesive 222 may be applied to the second surface 220 of the continuous elastic substrate 200a. The cutting device 304 separates the discrete elastic part 200 from the continuous elastic substrate 200a. Thus, as shown in FIGS. 7 and 8, the discrete elastic part 200 may also comprise corrugations 244 oriented to define corrugation lines 246 extending in the cross direction CD. In addition, the elastic part 200 includes a zone 240 of adhesive 222 that may cover corrugations 244 on the second surface 220 of the discrete elastic part 200, such as shown in FIGS. 7 and 8. Thus, the zone 240 of adhesive 222 may be separated into individual stripes of adhesive 222 when the central region 238 of the discrete elastic part 200 is stretched in the machine direction MD. The individual stripes of adhesive 222 may extend in the cross direction CD and may be separated from each other in the machine direction MD by areas of the second surface 220 of the elastic part 200 that do not include adhesive 222. In turn, the stretched central region 238 of the discrete elastic part 200 may then be bonded with the carrier substrate 202 with the stripes of adhesive 222.

It is also to be appreciated that the zone 240 of adhesive 222 may be applied to define various different shapes and sizes with respect to the discrete elastic part 200 and/or the carrier substrate 202. For example, as shown in FIG. 12, the zone 240 of adhesive 222 may extend in the cross direction CD to be coterminous with both the first and second longitudinal edges 214, 216 of the elastic part 200, and the zone 240 of adhesive 222 may define a length LAZ in the machine direction MD and a width WAZ in the cross direction CD. As such, the length LAZ of the zone 240 of adhesive 222 may extend the entire length LEP of the discrete elastic part 200 extending from the first longitudinal edge 214 to the second longitudinal edge 216. In some configurations such as shown in FIG. 12A, the length LAZ of the zone 240 of adhesive 222 may extend for less than the entire length LEP of the discrete elastic part 200. As such, the zone 240 of adhesive 222 may extend in the cross direction CD to be coterminous with the leading edge 230 and/or the trailing edge 232 of the elastic part 200 and may extend for length LAZ extending from either the first longitudinal edge 214 or the second longitudinal edge 216 of the elastic part 200.

In some examples, the zone 240 of adhesive 222 may not extend to either the leading edge 230 or the trailing edge 232 of the elastic part 200. In some configurations, more than one zone 240 of adhesive 222 may bond the elastic part 200 with the carrier substrate 202. For example, as shown in FIG. 12B, the laminate 204 may include a first zone 240a of adhesive and a second zone 240 b of adhesive 240 b that bond the elastic part 200 with the carrier substrate 202. Such zones 240a, 240 b of adhesive 222 may be separated from each other in the cross direction CD and/or the machine direction MD.

It is to be appreciated that the elastic part 200, carrier substrate 202, the zone 240 of adhesive 222, and the mechanical bonds and/or bond regions 242 may define various features with various sizes relative to each other. For example, as shown in FIG. 12, the elastic part 200 bonded with the carrier substrate 202 may define a width WEP extending in the cross direction CD from the leading edge 230 to the trailing edge 232. As such, the width WEP of the elastic part 200 may equal to or less than the WCS of the carrier substrate. The width WAZ of the zone 240 of adhesive 222 may be equal to or less than the width WEP of the elastic part 200.

As previously mentioned, the bonding device 340 may operate to mechanically bond the top and bottom regions 235, 237 of the elastic part 200 with the carrier substrate 202. As such, the mechanical bonds 242 may define bond zones wherein the laminate 204 of the elastic part 200 and carrier substrate 202 may or may not be elastic. For example, as shown in FIG. 12, a first bond zone BZ1 may extend from the mechanical bonds 242 located in the bottom region 237 of the elastic part 200 to leading edge 230 of the elastic part 200, and a second bond zone BZ2 may extend from the mechanical bonds 242 located in the top region 235 of the elastic part 200 to the trailing edge 232 of the elastic part 200. The first bond zone BZ1 may define a width WBZ1 in the cross direction CD, and the second bond zone BZ2 may define a width WBZ2 in the cross direction CD, wherein the widths WBZ1 and WBZ2 may be equal or different. As shown in FIG. 12, a corrugation zone CZ may be defined in the cross direction CD between the first bond zone BZ1 and the second bond zone BZ2 wherein the laminate 204 of the elastic part 200 and carrier substrate 202 is elastic. The corrugation zone CZ may define a width WCZ in the cross direction CD when in a fully stretched state, such as shown in FIG. 12.

In some configurations, the first bond zone BZ1 and/or the second bond zone BZ2 may be separated in the cross direction CD from the zone 240 of adhesive 222. For example, as shown in FIG. 12, the first bond zone BZ1 may be separated in the cross direction CD from the zone 240 of adhesive 222 to define a first gap zone GZ1, and the second bond zone BZ2 may be separated in the cross direction CD from the zone 240 of adhesive 222 to define a second gap zone GZ2. The first gap zone GZ1 may define a width WGZ1 in the cross direction CD, and the second gap zone GZ2 may define a width WGZ2 in the cross direction CD, wherein the widths WGZ1 and WGZ2 may be equal or different. In some configurations, the first width WGZ 1 and/or the second width WGZ2 may be from about 2 mm to about 4 mm. In some configurations, the first bond zone BZ1 and/or the second bond zone BZ2 may be coterminous with the zone 240 of adhesive 222, wherein the first width WGZ1 and/or the second width WGZ2 may be 0 mm. In some configurations, portions of the first bond zone BZ1 and/or the second bond zone BZ2 may be located inside the zone 240 of adhesive 222. In some configurations, portions of the first bond zone BZ1 and/or the second bond zone BZ2 may be located laterally outward from the leading edge 230 and/or the trailing edge 232.

As discussed above with reference to FIG. 2, the system 300 may include an adhesive applicator device 302 that may be configured to apply adhesive 222 to the continuous elastic substrate 200a upstream of the nip 310 between the knife roll 306 and anvil roll 308. In turn, the discrete elastic parts 200 separated from the continuous elastic substrate 200a may include a zone 240 of adhesive 222 that is adapted to adhesively bond the elastic part 200 with the carrier substrate 202. It is to be appreciated that the zone 240 of adhesive 222 may comprise adhesive 222 applied to the continuous elastic substrate 200a, the elastic part 200, and/or the carrier substrate 202 in various configurations and/or positions in the assembly process. For example, as shown in FIG. 2, the system 300 may include an adhesive applicator device 302a that may be configured to apply adhesive 222 to the discrete elastic part 200 at a position downstream of the nip 310 between the knife roll 306 and anvil roll 308. In another example, shown in FIG. 2, the apparatus 300 may include an adhesive applicator device 302b that deposits adhesive 222 onto the first surface 210 of the carrier substrate 202 to define the zone 240 of adhesive 222 that bonds the elastic part 200 with the carrier substrate 202. It is to be appreciated that the adhesive applicator device 302a may be configured to operate in addition to or in place of the adhesive applicators 302, 302b, and adhesive applicator device 302b may be configured to operate in addition to or in place of the adhesive applicators 302, 302a. It is also to be appreciated that the adhesive applicator devices 302a, 302b may be configured in various ways, such as the adhesive applicator 302 described above, such as for example, as a spray nozzle and/or a slot coating device. It is also to be appreciated that in some configurations, the discrete elastic parts 200 may be combined with the carrier substrate 202 with only mechanical bonds and without the use of adhesive.

In accordance with the above discussion with regard to the various shapes and sizes of the zones 240 of adhesive 222, it is to be appreciated that adhesive 222 may be applied to the continuous elastic substrate 200a and/or the carrier substrate 202 in various ways to define the zones 240 of adhesive 222. For example, as discussed above with reference to FIGS. 2 and 5, adhesive 222 may be applied to the continuous elastic substrate 200a to define a region 224 of adhesive 222 extending over discrete spaced apart regions in the machine direction MD and/or continuously in the cross direction CD. In another example, shown in FIG. 5B, the adhesive 222 may be applied to the second surface 220 of the continuous elastic substrate 200a in discrete patches 226 separated from each other in on the continuous elastic substrate 200a in the machine direction MD. In still another example, shown in FIG. 14, the adhesive 222 may be applied to the first surface 210 of the carrier substrate 202 in discrete patches 226 separated from each other on the carrier substrate 202 in the machine direction MD. It is to be appreciated that adhesive 222 may be applied to the continuous elastic substrate 200a, the elastic part 200, and/or the carrier substrate 202 in shapes and sizes that define the zones 240 of adhesive 222 that bond the elastic parts 200 and the carrier substrate 202 together. The discrete patches 226 of adhesive 222 may be separated from each other on the carrier substrate 202 in the machine direction MD by the pitch distance PD.

It is to be appreciated that the continuous elastic substrate 200a and the discrete elastic parts 200 herein may be configured in various ways and may include one or more elastic materials, such as for example, elastic film and/or strands. For example, the continuous elastic substrate 200a and the discrete elastic parts 200 may be configured as a single layer of elastic film. In some configurations, the continuous elastic substrate 200a and the discrete elastic parts 200 may be configured as a laminate of two more substrates. For example, the continuous elastic substrate 200a and the discrete elastic parts 200 may be configured as an elastic film bonded in between two or more nonwoven substrates and/or may be bonded with one or more nonwoven substrates. For example, the continuous elastic substrate 200a and the discrete elastic parts 200 may be configured as a bi-laminate with an elastic film bonded with a single nonwoven substrate. In another example, the continuous elastic substrate 200a and the discrete elastic parts 200 may be configured as an elastic film bonded between two or more substrates, wherein the substrates may comprise nonwovens. It is also to be appreciated that nonwoven substrates of the elastic substrate 200a and discrete elastic parts 200 may be of the same or different material and/or basis weights. In some configurations, one more nonwoven substrates of the elastic substrate 200a and discrete elastic parts 200 may be of the same or different material and/or basis weights as one more nonwoven substrates of the carrier substrate 202.

It is also to be appreciated that the continuous elastic substrate 200a and the discrete elastic parts 200 may be assembled in various ways, such as for example, as disclosed in U.S. Pat. Nos. 6,572,595; 6,830,800; 7,087,287; and 7,803,244; and U.S. Patent Publication Nos. 2018/0042778 A1; 2018/0042787 A1; 2018/0042779 A1; and 2018/0042780 A1, which are all incorporated by reference herein.

It is also to be appreciated that the processes and/or apparatuses herein may be configured with additional features, such as splicing operations, to help avoid having to stop assembly process operations in order to replenish material supplies. In some configurations, the apparatuses 300 discussed herein may be configured to operate with apparatuses that are configured to provide an uninterrupted supply of continuous elastic substrate 200a. For example, during operation, a replacement supply of a continuous elastic substrate 200a may be spliced to a current supply of continuous elastic substrate 200a being used in assembly operations before the current supply is completely depleted.

It is to be appreciated that various types of splicing operations may be used to replenish the supply of a continuous elastic substrate 200a. For example, some splicing operations may be configured to apply a strip of splicing tape to connect a replacement continuous elastic substrate 200a to a nearly depleted elastic substrate 200a to help avoid supply interruptions. As discussed above, the continuous elastic substrate 200a may advance through a cutting device 304 that separates the continuous elastic substrate 200a into discrete elastic parts 200. In addition, a transfer device 322 and/or bonding device 324 may further subject the discrete elastic parts 200 to machine directional stretching and/or bonding operations. However, some splicing tape material may not be stretchable and/or may not be conducive to bonding operations. In turn, discrete elastic parts 200 connected with splicing tape may undesirably disrupt operations of stretching and/or bonding processes. As such, some apparatuses 300 may be configured to remove discrete elastic parts 200 with splicing tape attached thereto from assembly operations before such undesired process disruptions may occur. In some examples, splicing operations may be configured to utilize stretchable splicing tape and/or other materials more conducive to various assembly operations to help prevent unintended assembly process disruptions and/or eliminate the need to remove elastic parts 200 with splicing tape attached thereto.

Some splicing operations may be configured to weld or otherwise bond a replacement supply of a continuous elastic substrate 200a to a current supply of continuous elastic substrate 200a being used in assembly operations without the need to use splicing tape. Such welding operations may utilize hot-wire or ultrasonic apparatuses to create a thermal splice. The thermal splice process may both cut and weld the materials together. In some configurations, thermal splices may be applied so as to maintain some stretch properties, which may allow discrete elastic parts 200 with such thermal splices to advance through cross directional stretching and/or bonding operations without disrupting such operations.

In some configurations, a visible mark may be utilized to reliably detect the splice for rejection. One way to mark the web is to utilize a marking system, such as for example, a printer, spray system, tape applicator, and the like, to apply an ink, pigment, tape, label and/or other visible mark to the moving continuous elastic substrate 200a after a splice is made. As such, the mark may be applied to the same surface of the continuous elastic substrate 200a for every splice. In order to automate such a system, the splice sequence may be configured to provide an output trigger signal to the marking system controller to initiate the marking process. Such a trigger output signal timing may occur when a clamping system releases the continuous elastic substrate 200a once the splice has been created. For a consistent web acceleration, a timing delay from the output trigger signal may be utilized to apply the mark as the continuous elastic substrate 200a accelerates after the splice sequence and the splice passes by. Alternatively, an encoder on the continuous elastic substrate 200a may be utilized with a known offset position to provide feedback to the marking control system to know when to apply the mark, allowing the mark to be applied on the splice weld or in close proximity to the weld. The visual mark may signal the splice location to operators and be detected downstream by a sensor to trigger a reject event.

Another way to mark the continuous elastic substrate 200a is to utilize a manual effort or a semi-automated marking system, such as for example, a printer, spray system, tape applicator, and the like to apply an ink, pigment, tape, label or other visible mark to a material roll before being loaded onto the splicer system. As such, the mark may be applied to the same web face for every splice. The web may be loaded into the splicing mechanism and the mark aligned such that when the splice is created, the mark would be split in half where the splice weld occurs. Thus, half of the mark adjacent may positioned to the splice position where the new roll begins. Alternatively, the mark may be aligned upstream in close proximity to where the splice weld will occur. In turn, the mark may be positioned immediately upstream of the splice weld. The visual mark would signal the splice location to operators and be detected downstream by a sensor to trigger a reject event.

Yet another way to mark the web would be to utilize a manual effort or a semi-automated marking system, such as for example, a printer, spray system, tape applicator, and the like to apply an ink, pigment, tape, label or other visible mark to the continuous elastic substrate 200a once the web has been loaded into the splicing mechanism. Depending on the web orientation in the splice mechanism, the mark may end up on either side of the web. A second marking system may be used on the opposite side of the web to ensure that both sides of the web are marked each time for consistency. The marking system may mark the web in a position such that when the splice is created, the mark would be split in half where the splice weld occurs. This would position half of the mark adjacent to the splice position where the new roll begins. Alternatively, the mark could be aligned upstream in close proximity to where the splice weld will occur. This would position the mark immediately upstream of the splice weld. The visual mark may signal the splice location to operators and be detected downstream by a sensor to trigger a reject event.

As discussed above, it is to be appreciated that the continuous elastic substrate 200a and the discrete elastic parts 200 herein may be configured in various ways and may include one or more elastic materials, such as for example, elastic film and/or strands. In some configurations, the continuous elastic substrate 200a and the discrete elastic parts 200 may comprise a single layer of elastic film. In some configurations, the continuous elastic substrate 200a and the discrete elastic parts 200 may comprise a laminate of two more substrates, such as an elastic film bonded with one or more nonwoven substrates. When the continuous elastic substrate 200a is configured to comprise an elastic film bonded with one or more consolidated nonwovens, a thermal splice may be configured to melt the layers of both film and nonwoven to create a weld that traps consolidations of the nonwoven materials. In turn, the cross directional stretching process may stretch the elastic part 200 such that the weld may also extend in the cross direction by partially failing a part of the weld that has trapped the consolidated nonwoven, sometimes referred to as "popping the weld." Depending on various splicing process parameters, such as for example weld time, dwell time, and quench time and various material properties, such as for example basis weight, fiber type, and plastic characteristics, the cross directional forces necessary to pop and stretch the weld may vary. In some examples, an ultrasonic splicing apparatus including a relatively sharp cutting anvil may be configured to produce a weld that has a relatively low cross directional force required to pop and stretch. In particular, a relatively smaller overall weld may be produced when a sharp angle of the anvil may penetrate through and burst fibers in the materials without causing a relatively large melt zone, while at the same time allowing the film to weld together, resulting in a splice that may be relatively easier to stretch in the cross direction CD with reduced and/or no popping required.

Tensile Test Method

Tensile Test Sample Preparation

(a) Tensile Test of a Laminate Substrate

A minimum of three specimens are collected and cut from the same portion of laminate material. Laminate machine direction is defined as the laminate web making process direction. If it cannot be clearly defined or it is unknown, then easy to stretch direction for the laminate is treated as Cross direction CD and the opposite direction is considered Machine direction MD. Sample is collected from the highest stretchy region of the laminate for CD and MD testing. If sample is uniformly stretchy across, or the highest stretchy region is not clearly defined then samples is collected from the center of the stretchy region. For tensile testing, specimen measuring at least 58 mm long in test direction and 25.4 mm long in the opposite direction is collected. For e.g., for MD tensile testing, specimen measuring at least 58 mm long in MD and 25.4 mm long in CD is collected from the laminate. If the larger sample cannot be prepared, smaller sample in the dimension of 17 mm in test direction and 10 mm in the opposite direction can be used. Test set-up for smaller sample should be selected as described in the table below.

(b) Tensile Test of Specimen from Absorbent Article

A minimum of three specimens are collected and cut from the same waistband portion of identical absorbent article products, and care should be taken to prevent damage of the specimen during the separation process. The Cross Direction (i.e., the maximum linear dimension) of the Waistband is defined as the easy to stretch direction, which will be lateral direction of the waistband on the product in most cases. A specimen measuring 17 mm in the lateral direction (CD) by 10 mm in the longitudinal direction (MD) of a given web is delicately cut from the highest stretchy region of the waistband for CD tensile testing. Sample in the dimension of 17 mm in test direction and 10 mm in the opposite direction is collected from the highest stretchy region of the waistband for MD testing or CD testing. If the waistband sample is uniformly stretchy across, or the highest stretchy region is not clearly defined then samples is collected from the center of the stretchy region. Test set-up for waistband sample should be selected as described in the table below.

Tensile Test Setup

A suitable tensile tester interfaced with a computer such as MTS model Alliance RT/1 with TestWorks 4[®] software or equivalent is used. The tensile tester is located in a temperature-controlled room at 22° C.±2° C. and 50±10% relative humidity. The instrument is calibrated according to the manufacturer's instructions. The data acquisition rate is set to at least 50 Hertz. The grips used for the test are wider than the sample and should allow testing at gage length preferred for the test. Grips having 50.8 mm width may be used. The grips are air actuated grips designed to concentrate the entire gripping force along a single line perpendicular to the direction of testing stress having one flat surface and an opposing face from which protrudes a half round (radius=6 mm, e.g., part number: 56-163-827 from MTS Systems Corp.) or equivalent grips, to minimize slippage of the specimen. The load cell is selected so that the forces measured are between 10% and 90% of the capacity of the load cell used. The initial distance between the lines of gripping force (gauge length) is set as per the table below. The load reading on the instrument is zeroed to account for the mass of the fixture and grips.

The specimen is mounted into the grips in a manner such that there is no slack and the load measured is between 0.00 N and 0.02 N. The specimen is mounted in the center of the grips, such that the specimen direction of stretching is parallel to the applied tensile stress.

Tensile Test

The instrument is set up and the specimen mounted as described in the Tensile Test Setup above. The tensile test is initiated and the specimen is extended at speed described in the table below, with a data acquisition rate of at least 50 Hertz, until the specimen breaks, typically 50-1000% strain. The % elongation (used here interchangeably with % strain) is calculated from the length between grip lines L, and initial gauge length, L0, using the following formula:

$$\% \text{ Strain OR } \% \text{ Elongation} = \frac{(L - L_0)}{L_0} \times 100$$

| | Specimen Length in the test direction | Gage Length to be used for the test | Test speed to be used for the test | Strain Rate (1/sec) - Test speed/gage length |
|---|---|---|---|---|
| Small Sample | 17 mm | 10 mm | 100 mm/min | 0.1667 (1/sec) |
| Regular Sample | 58 mm | 50.8 mm | 508 mm/min | 0.1667 (1/sec) |

Three specimens of each set are measured, and the arithmetic average of tensile force at 5% elongation (N), and Peak Elongation (or strain) at break (%) are recorded. Peak Elongation is defined as % elongation at Peak (maximum) Load on the tensile curve.

COMBINATIONS

A1. A method of assembling absorbent articles, the method comprising steps of: advancing a carrier substrate at a first speed in a machine direction, the carrier substrate comprising a first longitudinal edge and a second longitudinal edge separated from the first longitudinal side in a cross direction; advancing a continuous elastic substrate at a second speed in the machine direction, the continuous elastic substrate comprising a first longitudinal edge and a second longitudinal edge separated from the first longitudinal edge in the cross direction, wherein the continuous elastic substrate is stretchable in the machine direction; stretching the central region of the discrete elastic part in the machine direction; cutting an elastic part from the continuous elastic substrate, the elastic part comprising a first end region and a second end region separated from the first end region in the machine direction by a central region; changing a speed of the elastic part from the second speed to the first speed; rotating the discrete elastic part such that the second end region is separated from the first end region in the cross direction by the central region; positioning the elastic part on the carrier substrate such that the stretched central region extends in the cross direction between the first and second longitudinal edges of the carrier substrate; adhesively bonding the stretched central region of the elastic part with the carrier substrate; and mechanically bonding the first end region and the second end region of the elastic part with the carrier substrate.

A2. The method according to paragraph A1, further comprising a step of dividing the elastic part into a first waistband and a second waistband by cutting the carrier substrate along the cross direction through the elastic part.

A3. The method according to paragraphs A1 or A2, wherein the step of adhesively bonding further comprises applying adhesive to the central region of the elastic part without applying adhesive to the first end region and the second end region of the elastic part.

A4. The method according to paragraph A3, wherein the step of adhesively bonding further comprises adhesively bonding the stretched central region of the elastic part with the carrier substrate without adhesively bonding the first end region and the second end region of the elastic part with the carrier substrate.

A5. The method according to any one of paragraphs A1-A4, wherein the carrier substrate comprises a topsheet substrate comprising a first surface and an opposing second surface, and wherein the elastic part comprises a first surface and an opposing second surface.

A6. The method according to paragraph A5, wherein the step of positioning the elastic part on the carrier substrate further comprises positioning the second surface of the elastic part in a facing relationship with the first surface of the topsheet substrate.

A7. The method according to paragraph A6, further comprising steps of: positioning an absorbent core on the second surface of the topsheet substrate; and bonding a backsheet substrate with the topsheet substrate with the absorbent core sandwiched between the topsheet substrate and the backsheet substrate.

A8. The method according to paragraph A5, further comprising a step of bonding leg gasketing assemblies with the topsheet substrate.

A9. The method according to paragraph A8, wherein the step of positioning the second surface of the elastic part in a facing relationship with the first surface of the topsheet substrate is performed after the step of bonding leg gasketing assemblies with the topsheet substrate.

A10. The method according to paragraph A9, wherein a portion of the leg gasketing assemblies are positioned between the elastic part and the topsheet substrate.

A11. The method according to any one of paragraphs A1-A10, wherein the continuous elastic substrate comprises elastic strands.

A12. The method according to paragraph A11, wherein the continuous elastic substrate comprises a nonwoven bonded with the elastic strands.

A13. The method according to any one of paragraphs A1-A12, further comprising steps of applying adhesive to the elastic part; and rotating the elastic part around an axis of rotation wherein the adhesive is facing radially inward.

A14. The method according to any one of paragraphs A1-A13, wherein the stretching step comprises accelerating the central region of the discrete elastic part from the second speed to a third speed greater than the second speed.

A15. The method according to paragraph A14, wherein the stretching step further comprises transferring the discrete elastic part to an outer surface of a rotating anvil that rotates at the third speed.

A16. The method according to any one of paragraphs A1-A15, wherein the rotating step comprises receiving, at a rotational mechanism, the stretched elastic part in the machine direction and rotating, with the rotational mechanism, the stretched elastic part such that a longitudinal axis of the stretched elastic part rotates from being aligned in the machine direction to being aligned in the cross direction.

A17. The method according to paragraph A16, wherein the rotational mechanism comprises a plurality of axles and a plate attached to an end of each axle, wherein the axles are configured to rotate about a first rotational axis and wherein each plate is configured to rotate about a second axis defined by the respective axle, wherein the stretched elastic plate is received by one of the plates and wherein the rotating step comprises rotating the plate about the second axis.

B1. A method of assembling absorbent articles, the method comprising steps of: providing an elastic part comprising a first surface and an opposing second surface, the elastic part further comprising a first end region and a second end region separated from the first end region in a machine direction by a central region; providing a zone of adhesive positioned on the second surface of the elastic part; advancing the elastic part in a machine direction on a first roll, wherein the second surface is facing radially outward; stretching the central region of the elastic part in the machine direction; transferring the first end region and the second end region of the elastic part from the first roll to a rotatable transfer device, wherein the second surface of the elastic part is facing radially inward; rotating the discrete elastic part such that the second end region is separated from the first end region in the cross direction by the central region; transferring the stretched elastic part to a second roll, wherein the second surface of the elastic part is facing radially outward; advancing a carrier substrate adjacent the second roll, the carrier substrate comprising a first longitudinal edge and a second longitudinal edge separated from the first longitudinal side in the cross direction; advancing the elastic part from the second roll to the carrier substrate such that the stretched central region extends in the cross direction between the first and second longitudinal edges of the carrier substrate; adhesively bonding the stretched central region of the elastic part with the carrier substrate; and mechanically bonding the first end region and the second end region of the elastic part with the carrier substrate.

B2. The method according to paragraph B1, wherein the second roll comprises a pattern roll.

C1. A method of assembling absorbent articles, the method comprising steps of: advancing a carrier substrate at a first speed in a machine direction, the carrier substrate comprising a first longitudinal edge and a second longitudinal edge separated from the first longitudinal edge in a cross direction; advancing a continuous elastic substrate at a second speed in the machine direction, the continuous elastic substrate comprising a first longitudinal edge and a second longitudinal edge separated from the first longitudinal edge in the cross direction, wherein the continuous elastic substrate is stretchable in the machine direction; cutting an elastic part from the continuous elastic substrate, the elastic part comprising a first end region and a second end region separated from the first end region in the machine direction by a central region; changing a speed of the elastic part from the second speed to the first speed; stretching the central region of the discrete elastic part in the machine direction; rotating the discrete elastic part such that the stretched central region is oriented in the cross direction; and positioning the elastic part on the carrier substrate such that the stretched central region extends in the cross direction between the first and second longitudinal edges of the carrier substrate.

C2. The method according to paragraph C1, further comprising dividing the elastic part into a first waistband and a second waistband by cutting the carrier substrate along the cross direction through the elastic part.

C3. The method according to any one of paragraphs C1-C2, wherein the stretching step comprises accelerating the central region of the discrete elastic part from the second speed to a third speed greater than the second speed.

C4. The method according to paragraph C3, wherein the stretching step further comprises attaching the discrete elastic part to an outer surface of a rotating anvil that rotates at the third speed.

C5. The method according to any one of paragraphs C1-C4, wherein the rotating step comprises receiving, at a rotational mechanism, the stretched elastic part in the machine direction and rotating, with the rotational mechanism, the stretched elastic part such that a longitudinal axis of the stretched elastic part rotates from being aligned in the machine direction to being aligned in the cross direction.

C6. The method according to paragraph C5, wherein the rotational mechanism comprises a plurality of axles and a plate attached to an end of each axle, wherein the axles are configured to rotate about a first rotational axis and wherein each plate is configured to rotate about a second axis defined by the respective axle, wherein the stretched elastic plate is received by one of the plates and wherein the rotating step comprises rotating the plate about the second axis.

C7. The method according to any one of paragraphs C1-C6, wherein the first waistband defines a first length in the machine direction and the second waistband defines a second length in the machine direction, wherein the first length is different than the second length.

Bio-Based Content for Components

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260™, SHE150™, or SGM9450F™ (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Recycle Friendly and Bio-Based Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. Appl. Publ. No. 2019/0192723, published on June 27, 2019.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

**Claims**

1.  A method of assembling absorbent articles, the method comprising steps of:

        advancing a carrier substrate (202) at a first speed in a machine direction, the carrier substrate (202) comprising a first longitudinal edge (206) and a second longitudinal edge (208) separated from the first longitudinal side (206) in a cross direction;
        advancing a continuous elastic substrate (200a) at a second speed in the machine direction, the continuous elastic substrate (200a) comprising a first longitudinal edge (214) and a second longitudinal edge (216) separated from the first longitudinal edge (214) in the cross direction, wherein the continuous elastic substrate (200a) is stretchable in the machine direction;
        cutting an elastic part (200) from the continuous elastic substrate (200a), the elastic part (200) comprising a first end region (235) and a second end region (237) separated from the first end region (235) in the machine direction by a central region (238);
        stretching the central region (238) of the discrete elastic part (200) in the machine direction;
        changing a speed of the elastic part (200) from the second speed to the first speed;

rotating the discrete elastic part (200) such that the second end region (237) is separated from the first end region (235) in the cross direction by the central region (238);

positioning the elastic part (200) on the carrier substrate (202) such that the stretched central region (238) extends in the cross direction between the first and second longitudinal edges (206, 208) of the carrier substrate (202);

adhesively bonding the stretched central region (238) of the elastic part (200) with the carrier substrate (202); and

mechanically bonding the first end region (235) and the second end region (237) of the elastic part (200) with the carrier substrate (202).

2. The method according to claim 1, further comprising a step of dividing the elastic part (200) into a first waistband (158) and a second waistband (158) by cutting the carrier substrate (202) along the cross direction through the elastic part (200).

3. The method according to either claim 1 or 2, wherein the step of adhesively bonding further comprises applying adhesive (222) to the central region of the elastic part (200) without applying adhesive to the first end region (235) and the second end region (237) of the elastic part (200).

4. The method according to claim 3, wherein the step of adhesively bonding further comprises adhesively bonding the stretched central region (238) of the elastic part (200) with the carrier substrate (202) without adhesively bonding the first end region (235) and the second end region (237) of the elastic part (200) with the carrier substrate (202).

5. The method according any of the preceding claims, wherein the carrier substrate (202) comprises a topsheet substrate (138) comprising a first surface and an opposing second surface, and wherein the elastic part (200) comprises a first surface and an opposing second surface.

6. The method according to claim 5, wherein the step of positioning the elastic part (200) on the carrier substrate (202) further comprises positioning the second surface of the elastic part (200) in a facing relationship with the first surface of the topsheet substrate (138).

7. The method according to claim 6, further comprising steps of: positioning an absorbent core (140) on the second surface of the topsheet substrate (138); and bonding a backsheet substrate (136) with the topsheet substrate (138) with the absorbent core (140) sandwiched between the topsheet substrate (138) and the backsheet substrate (136).

8. The method according to claim 5, further comprising a step of bonding leg gasketing assemblies (156) with the topsheet substrate (138).

9. The method according to claim 8, wherein the step of positioning the second surface of the elastic part (200) in a facing relationship with the first surface of the topsheet substrate (138) is performed after the step of bonding leg gasketing assemblies (156) with the topsheet substrate (138).

10. The method according to any of the preceding claims, wherein the continuous elastic substrate (200a) comprises elastic strands, and wherein the continuous elastic substrate (200a) comprises a nonwoven bonded with the elastic strands.

11. The method according to any of the preceding claims, further comprising steps of applying adhesive (222) to the elastic part (200); and rotating the elastic part (200) around an axis of rotation wherein the adhesive (222) is facing radially inward.

12. The method according to any of the preceding claims, wherein the stretching step comprises accelerating the central region (238) of the discrete elastic part (200) from the second speed to a third speed greater than the second speed.

13. The method according to claim 14, wherein the stretching step further comprises transferring the discrete elastic part (200) to an outer surface (318) of a rotating anvil (308) that rotates at the third speed.

14. The method according to any of the preceding claims, wherein the rotating step comprises receiving, at a rotational mechanism (326), the stretched elastic part (200) in the machine direction and rotating, with the rotational mechanism (326), the stretched elastic part (200) such that a longitudinal axis of the stretched elastic part (200) rotates from being aligned in the machine direction to being aligned in the cross direction.

**15.** The method of claim 14, wherein the rotational mechanism (326) comprises a plurality of axles (374) and a plate (376) attached to an end of each axle (374), wherein the axles (374) are configured to rotate about a first rotational axis and wherein each plate (376) is configured to rotate about a second axis defined by the respective axle, wherein the stretched elastic part (200) is received by one of the plates (376) and wherein the rotating step comprises rotating the plate about the second axis.

Figure 1A

Figure 1B

Figure 2

Figure 2B

Figure 2C

EP 4 364 707 A1

Figure 3

Figure 2A

208

210

202

156

CD

W_CS

206

156

S1

MD

## Figure 3A

216   200a   220

CD

W_ES

MD

214

## Figure 4

MD

244  246  200a  220  216

4A1  4A1

W$_{ES}$  CD

244  246  214

Figure 4A

MD

244  246  244  246  220  200a

244  246  244  246  218

Figure 4A1

Figure 5

Figure 5A

EP 4 364 707 A1

Figure 5B

Figure 6

Figure 10

Figure 7

Figure 8

300

CD

316

306

304

312

314

310

308

318

320

W

9A

9A

377

376

322,
326

L_EP2

11

11

348

324

340

346

Figure 9

237    222    235

240    377

379    376    381

## Figure 9A

216    220    239    222,    240    CD    S3    238    200

237    235

230    232    W

214    $L_{EP2}$    MD

## Figure 10A

EP 4 364 707 A1

Figure 11

Figure 11A

Figure 13

Figure 14

Figure 12

Figure 12A

Figure 12B

Figure 12C

EP 4 364 707 A1

Figure 12D

Europäisches
Patentamt

European
Patent Office

Office européen
des brevets

# EUROPEAN SEARCH REPORT

Application Number

EP 23 20 6916

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/128366 A1 (SCHNEIDER UWE [US] ET AL) 6 May 2021 (2021-05-06) | 1-13 | INV.<br>A61F13/15 |
| Y | * claims 1-20 *<br>* figures 1-10 *<br>* paragraph [0108] * | 1-15 | A61F13/49 |
| X | US 2020/375807 A1 (SCHNEIDER UWE [US] ET AL) 3 December 2020 (2020-12-03) | 1-13 | |
| Y | * claims 1-35 *<br>* figures 1-10 *<br>* paragraphs [0011] - [0014], [0104] * | 1-15 | |
| Y | US 9 737 442 B2 (PROCTER & GAMBLE [US]) 22 August 2017 (2017-08-22)<br>* claims 1-17; figures 6-8 * | 1-15 | |

| | |
|---|---|
| | TECHNICAL FIELDS SEARCHED (IPC)<br><br>A61F |

The present search report has been drawn up for all claims

| Place of search | Date of completion of the search | Examiner |
|---|---|---|
| Munich | 12 March 2024 | Beins, Ulrika |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
..............................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 20 6916

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2024

| Patent document cited in search report | | | Publication date | Patent family member(s) | | | Publication date |
|---|---|---|---|---|---|---|---|
| US 2021128366 | A1 | | 06-05-2021 | CN | 114585337 | A | 03-06-2022 |
| | | | | EP | 4054497 | A1 | 14-09-2022 |
| | | | | EP | 4054501 | A1 | 14-09-2022 |
| | | | | EP | 4279045 | A2 | 22-11-2023 |
| | | | | JP | 7377969 | B2 | 10-11-2023 |
| | | | | JP | 2022553961 | A | 27-12-2022 |
| | | | | US | 2021128366 | A1 | 06-05-2021 |
| | | | | US | 2021128369 | A1 | 06-05-2021 |
| | | | | WO | 2021092606 | A1 | 14-05-2021 |
| | | | | WO | 2021092607 | A1 | 14-05-2021 |
| US 2020375807 | A1 | | 03-12-2020 | CN | 114025727 | A | 08-02-2022 |
| | | | | EP | 3975958 | A1 | 06-04-2022 |
| | | | | EP | 3975962 | A1 | 06-04-2022 |
| | | | | JP | 2022534905 | A | 04-08-2022 |
| | | | | US | 2020375807 | A1 | 03-12-2020 |
| | | | | US | 2020375815 | A1 | 03-12-2020 |
| | | | | US | 2020375816 | A1 | 03-12-2020 |
| | | | | US | 2024050285 | A1 | 15-02-2024 |
| | | | | WO | 2020242714 | A1 | 03-12-2020 |
| | | | | WO | 2020242715 | A1 | 03-12-2020 |
| US 9737442 | B2 | | 22-08-2017 | EP | 3302385 | A1 | 11-04-2018 |
| | | | | US | 2016354258 | A1 | 08-12-2016 |
| | | | | US | 2017290712 | A1 | 12-10-2017 |
| | | | | WO | 2016196744 | A1 | 08-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63422467
- US 4324246 A
- US 4463045 A
- US 4342314 A
- US 4556146 A
- US 4589876 A
- US 4687478 A
- US 4950264 A
- US 5009653 A
- US 5267992 A
- US 6004893 A
- US 20070219521 A1, Hird
- US 20110139658 A1, Hird
- US 20110139657 A1, Hird
- US 20110152812 A1, Hird
- US 20110139662 A1, Hird
- US 20110139659 A1, Hird
- US 5167897 A
- US 5360420 A
- US 5599335 A
- US 5643588 A
- US 5674216 A
- US 5702551 A
- US 5968025 A
- US 6107537 A
- US 6118041 A
- US 6153209 A
- US 6410129 B
- US 6426444 B
- US 6586652 B
- US 6627787 B
- US 6617016 B
- US 6825393 B
- US 6861571 B
- US 20130072887 A1
- US 20130211356 A1
- US 20130306226 A1
- US 4940464 A
- US 5092861 A
- US 5246433 A
- US 5569234 A
- US 5897545 A
- US 5957908 A
- US 6120487 A
- US 6120489 A
- US 7569039 B
- US 20030233082 A1
- US 20050107764 A1
- US 20120061016 A1
- US 20120061015 A1
- US 20130255861 A1
- US 20130255862 A1
- US 20130255863 A1
- US 20130255864 A1
- US 20130255865 A1
- US 5628097 A
- US 5916661 A
- US 6545197 B
- US 6107539 A
- US 4610678 A
- US 4673402 A
- US 4888231 A
- US 4834735 A
- US 5562646 A
- US 5669894 A
- US 6790798 B
- US 20040158212 A1
- US 20040097895 A1
- US 3860003 A
- US 4909803 A
- US 4695278 A
- US 4795454 A
- US 4704115 A
- US 20090312730 A1
- US 4515595 A
- US 5151092 A
- US 7371302 B
- US 3848594 A
- US 4662875 A
- US 4846815 A
- US 4894060 A
- US 4946527 A
- US 5221274 A
- US 6251097 B
- US 6669618 B
- US 6432098 B
- US 20070078427 A1
- US 20070093769 A1
- US 5735840 A
- US 5928212 A
- US 8186296 B
- US 9265672 B
- US 9248054 B
- US 9295590 B
- US 20140148773 A1
- US 7587966 B
- US 9168182 B
- US 9737442 B

- US 20210267812 A
- US 20220008256 A
- EP 2260813 B1
- US 4854984 A
- US 6248195 B
- US 8778127 B
- US 9005392 B
- US 9962297 B
- US 10052237 B
- US 3113225 A
- US 3562041 A
- US 3733238 A
- US 5110403 A
- US 6036796 A
- US 6508641 B
- US 6645330 B
- US 8608720 B
- US 20170246052 A1
- US 6572595 B
- US 6830800 B
- US 7087287 B
- US 7803244 B
- US 20180042778 A1
- US 20180042787 A1
- US 20180042779 A1
- US 20180042780 A1
- US 20070219521
- US 8703450 B
- US 9630901 B
- US 9822197 B
- US 20110139657
- US 20110139658 A
- US 20110152812 A
- US 20160206774 A
- US 9169366 B
- US 20190192723 A